# EUROPEAN PATENT APPLICATION

(11) **EP 4 311 474 A1**
(43) Date of publication of application: **31.01.2024**
(21) Application number: 23188317.4
(22) Date of filing: 28.07.2023
(51) Int. Cl.: A61B 3/00

(54) **OPHTHALMIC APPARATUS AND OPERATION UNIT**

(30) Priority: 29.07.2022 JP 2022122322; 29.07.2022 JP 2022122323; 06.06.2023 JP 2023093544; 27.07.2023 JP 2023122302
(71) Applicant: Nidek Co., Ltd., Gamagori-shi, Aichi 443-0038 (JP)
(72) Inventor: AOKI, Kenji, Gamagori, Aichi (JP); HAMAGUCHI, Koji, Gamagori, Aichi (JP); NAKAMURA, Kenji, Gamagori, Aichi (JP); UMANO, Hiroyuki, Gamagori, Aichi (JP); YOSHIMURA, Yuuto, Gamagori, Aichi (JP)
(74) Representative: Hoefer & Partner Patentanwälte mbB

(57) **Abstract**

A control unit of an ophthalmic apparatus finely drives and roughly drives a drive unit of an optometry unit. In a case where a tilt operation of tilting the operation stick within a predetermined range is detected by an operation detection unit, the control unit finely drives the drive unit by controlling the drive unit in response to the detected tilt operation, and finely changes a position of the optometry unit. In a case where at least one of a tilt operation of tilting the operation stick over a predetermined range and an operation of a rough movement operation unit is detected by the operation detection unit, the control unit roughly drives the drive unit by controlling the drive unit in response to the detected operation, and roughly changes the position of the optometry unit.

## Description

### TECHNICAL FIELD

The present disclosure relates to an ophthalmic apparatus for examining a subject eye, and an operation unit operated for changing a relative position of an optometry unit of the ophthalmic apparatus with respect to the subject eye.

### BACKGROUND

Various ophthalmic apparatuses for examining a subject eye (for example, an eye refractive power measurement apparatus, a corneal curvature measurement apparatus, an intraocular pressure measurement apparatus, a fundus camera, an OCT apparatus, a laser scanning optometry apparatus (SLO), and the like) are known. Examination of the subject eye by multiple ophthalmic apparatuses needs to be performed in a state where the relative position between the subject eye and the optometry unit is adjusted to a proper position.

An ophthalmic apparatus is also known that allows at least a part of the apparatus to be finely and roughly moved in order to efficiently adjust the relative position between the subject eye and the optometry unit. A fine movement is a small (or slow) movement of at least a part of the apparatus in order to finely adjust the relative position. A rough movement is a movement of at least a part of the apparatus larger (or faster) than a fine movement in order to roughly adjust the relative position.

For example, an ophthalmic apparatus described in JP2014-023960A finely moves an optometry unit in a case where an operation stick is tilted within a certain tilting range, and roughly moves the optometry unit in a case where a push button on the top of the operation stick is operated. In addition, there is also known an ophthalmic apparatus or the like that finely moves an optometry unit when a tilting range of an operation stick is within a predetermined range, and roughly moves the optometry unit when the tilting range of the operation stick exceeds a predetermined range.

In the ophthalmic apparatus in which a rough movement instruction is input by an operation different from the tilt operation of tilting the operation stick, it is possible to input the rough movement instruction without tilting the operation stick, but since an operation part differs between the fine movement and the rough movement, switching between the fine movement and the rough movement tends to be complicated. On the other hand, in the ophthalmic apparatus that roughly moves the optometry unit when the tilting range of the operation stick exceeds a predetermined range, both a fine movement instruction and a rough movement instruction can be input using the operation stick, but when inputting the rough movement instruction, an operation of greatly tilting the operation stick is required. Therefore, an ophthalmic apparatus is desired that allows both the fine movement instruction and the rough movement instruction to be input more easily and appropriately.

### SUMMARY

A typical object of the present disclosure is to provide an ophthalmic apparatus and an operation unit that allow more easily and appropriately input of both a fine movement instruction and a rough movement instruction for an optometry unit.
(1) An ophthalmic apparatus includes an optometry unit configured to examine a subject eye, a drive unit configured to change a relative position of the optometry unit with respect to the subject eye, an operation stick supported to be tiltable in any direction, a rough movement operation unit operated by an examiner to roughly move the optometry unit, an operation detection unit configured to detect an operation of tiling the operation stick and the rough movement operation unit, and a control unit configured to control the drive unit, in a case where the operation detection unit detects a tilt operation of tilting the operation stick within a predetermined range, in response to the detected tilt operation, to finely drive the drive unit and finely change a position of the optometry unit, and control the drive unit, in a case where the operation detection unit detects at least one of a tilt operation of tilting the operation stick over the predetermined range and an operation of the rough movement operation unit, in response to the detected operation, to roughly drive the drive unit and roughly change the position of the optometry unit.
(2) The ophthalmic apparatus according to the above-described (1),
   in which the rough movement operation unit is an annular member disposed on an outer periphery of the operation stick formed in a rod shape to surround the operation stick, and
   in which the rough movement operation unit is supported to be slidable in two-dimensional directions.
(3) The ophthalmic apparatus according to the above-described (1) or (2),
   in which in a case where a tilt angle of the operation stick is within the predetermined range, the operation stick is tilted to be independent of the rough movement operation unit, and
   in which in a case where the tilt angle of the operation stick exceeds the predetermined range, the operation stick is tilted while contacting the rough movement operation unit to operate the rough movement operation unit.
(4) The ophthalmic apparatus according to the above-described (3),
   in which the operation detection unit includes a rough movement operation detection unit that detects that the rough movement operation unit is operated, and
   in which both the tilt operation of tilting the operation stick over the predetermined range and the operation of the rough movement operation unit are detected by the rough movement operation detection unit.
(5) The ophthalmic apparatus according to the above-described (3) or (4), further including:
   a biasing unit configured to bias the rough movement operation unit toward an initial position where the rough movement operation unit is in contact with the operation stick in a state where the tilt angle reaches a boundary of the predetermined range,
   in which when the tilt operation of tilting the operation stick over the predetermined range is completed, the biasing unit moves the rough movement operation unit toward the initial position to return the tilt angle of the operation stick being in contact with the rough movement operation unit within the predetermined range.
(6) The ophthalmic apparatus according to any one of the above-described (1) to (5),
   in which in a case where an operation amount of the rough movement operation unit exceeds a specified amount, the operation detection unit detects that the rough movement operation unit is operated.
(7) The ophthalmic apparatus according to any one of the above-described (1) to (6), further including:
   a guide unit configured to guide a movement of the rough movement operation unit on a slide plane which is a two-dimensional plane on which the rough movement operation unit is slid, in which one direction on the slide plane is defined as an X direction, a direction intersecting the X direction on the slide plane is defined as a Z direction, and a direction intersecting the slide plane is defined as a Y direction,
   in which the guide unit includes:
      an X-direction linear guide unit linearly extending in the X direction;
      a Z-direction linear guide unit linearly extending in the Z direction;
      an X-direction slide unit attached to the X-direction linear guide unit and relatively sliding in the X direction with respect to the X-direction linear guide unit; and
      a Z-direction slide unit attached to the Z-direction linear guide unit and relatively sliding in the Z direction with respect to the Z-direction linear guide unit,
   in which the X-direction linear guide unit slides in the X direction with respect to the X-direction slide unit to guide a movement of the rough movement operation unit in the X direction, and
   in which the Z-direction slide unit slides in the Z direction with respect to the Z-direction linear guide unit to guide a movement of the rough movement operation unit in the Z direction.
(8) The ophthalmic apparatus according to the above-described (7),
   in which a pair of the X-direction linear guide units are disposed at each side of a +Z direction and a -Z direction than a central portion in an XZ direction of the rough movement operation unit, and extend linearly in the X direction in parallel to each other,
   in which a pair of the Z-direction linear guide units are disposed at each side of a +X direction and a -X direction than the central portion in the XZ direction of the rough movement operation unit, and extend linearly in the Z direction in parallel to each other,
   in which a pair of the X-direction slide units are attached to each of the pair of X-direction linear guide units, and
   in which a pair of the Z-direction slide units are attached to each of the pair of Z-direction linear guide units.
(9) The ophthalmic apparatus according to the above-described (7) or (8),
   in which the X-direction linear guide unit is provided with an X-direction biasing unit configured to bias a position of the X-direction linear guide unit with respect to the X-direction slide unit toward an initial X position where the rough movement operation unit is not operated in the X direction, and
   in which the Z-direction slide unit is provided with a Z-direction biasing unit configured to bias a position of the Z-direction slide unit with respect to the Z-direction linear guide unit toward an initial Z position where the rough movement operation unit is not operated in the Z direction.
(10) The ophthalmic apparatus according to any one of the above-described (7) to (9),
   in which the X-direction linear guide unit and the Z-direction linear guide unit are disposed on the same XZ plane.
(11) The ophthalmic apparatus according to any one of claims (7) to (10),
   in which the rough movement operation unit is connected with the Z-direction slide unit, in which the Z-direction slide unit moves on an XZ plane in synchronization with a movement of the rough movement operation unit on the XZ plane, and
   in which the X-direction linear guide unit and the Z-direction linear guide unit are connected with each other to integrally move in the X direction.
(12) An operation unit provided by a typical embodiment of the present disclosure is an operation unit operated by an examiner to change a relative position of an optometry unit of an ophthalmic apparatus with respect to a subject eye, the operation unit including an operation stick supported to be tiltable in any direction, a rough movement operation unit operated by the examiner to roughly move the optometry unit, and an operation detection unit configured to detect an operation of the operation stick and the rough movement operation unit, in which in a case where the operation detection unit detects a tilt operation of tilting the operation stick within a predetermined range, a position of the optometry unit is finely changed in response to the detected tilt operation, and in a case where the operation detection unit detects at least one of a tilt operation of tilting the operation stick over the predetermined range and an operation of the rough movement operation unit, the position of the optometry unit is roughly changed in response to the detected operation.

According to the ophthalmic apparatus and the operation unit according to the present disclosure, both the fine movement instruction and the rough movement instruction for the optometry unit are input more easily and appropriately.

### BRIEF DESCRIPTION OF DRAWINGS

FIG. 1 is a right side view illustrating an appearance of an ophthalmic apparatus 1.
FIG. 2 is a view illustrating an optical system and a control system of the ophthalmic apparatus 1.
FIG. 3 is a perspective view of an operation unit 60 according to a first embodiment as viewed diagonally from the rear right.
FIG. 4 is a cross-sectional view taken along the line A-A in FIG. 3.
FIG. 5 is a view illustrating a state where an operation stick 61 is tilted in the +X direction over a predetermined range from the state illustrated in FIG. 3.
FIG. 6 is a flowchart of relative position adjustment processing performed by the ophthalmic apparatus 1.
FIG. 7 is a perspective view of an operation unit 80 according to a second embodiment as viewed diagonally from the front right.
FIG. 8 is a cross-sectional view taken along the line A-A in FIG. 7.
FIG. 9 is a plan view of the operation unit 80 according to the second embodiment.
FIG. 10 is a view comparing plan views of the operation unit 80 according to the second embodiment in cases where (0) the rough movement operation unit 70 is disposed at (0) an initial position, (1) the rough movement operation unit 70 is moved in +X direction, (2) the rough movement operation unit 70 is moved in +Z direction, and (3) the rough movement operation unit 70 is moved in +X direction and +Z direction.

### DETAILED DESCRIPTION

### <Overview>

An ophthalmic apparatus exemplified in the present disclosure is provided with an optometry unit, a drive unit, an operation stick, a rough movement operation unit, an operation detection unit, and a control unit. The optometry unit is used for examining a subject eye. The drive unit changes the relative position of the optometry unit with respect to the subject eye. The operation stick is supported so as to be tiltable in any direction. The rough movement operation unit is operated by the examiner for roughly moving the optometry unit. The operation detection unit detects operations of the operation stick and the rough movement operation unit. The control unit performs fine driving and rough driving of the drive unit. In a case where a tilt operation of tilting the operation stick within a predetermined range is detected by the operation detection unit, the control unit finely drives the drive unit by controlling the drive unit in response to the detected tilt operation, and finely changes the position of the optometry unit. In a case where at least one of a tilt operation of tilting the operation stick over a predetermined range and an operation of the rough movement operation unit is detected by the operation detection unit, the control unit roughly drives the drive unit by controlling the drive unit in response to the detected operation, and roughly changes the position of the optometry unit. As described above, a fine movement means moving the optometry unit smaller (or slower) than a rough movement in order to finely adjust the relative position between the subject eye and the optometry unit. For example, the control unit may control the optometry unit to move smaller than the rough movement, by allowing the optometry unit to move within a movable range which is smaller than a movable range for the rough movement. A movement speed of the optometry unit during the fine movement may be different from a movement speed of the optometry unit during the rough movement. For example, the movement speed of the optometry unit during the fine movement may be lower than the movement speed of the optometry unit during the rough movement. The movement speed of the optometry unit during the fine movement may be equal to the movement speed of the optometry unit during the rough movement. The rough movement means moving the optometry unit larger (or faster) than the fine movement in order to roughly adjust the relative position between the subject eye and the optometry unit. For example, the control unit may control the optometry unit to move larger than the fine movement, by allowing the optometry unit to move within a movable range which is larger than a movable range for the fine movement. For example, the control unit may control the optometry unit to move larger than the fine movement, by continuing the movement of the optometry unit while an examiner inputs a direction for the rough movement, regardless of the movable range for the fine movement. The movement speed of the optometry unit during the rough movement may be different from the movement speed of the optometry unit during the fine movement. For example, the movement speed of the optometry unit during the rough movement may be higher than the movement speed of the optometry unit during the fine movement. The movement speed of the optometry unit during the rough movement may be equal to the movement speed of the optometry unit during the fine movement.

In the ophthalmic apparatus according to the present disclosure, the examiner can input a rough movement instruction for the optometry unit even when either a tilt operation of tilting the operation stick over a predetermined range or an operation of the rough movement operation unit is performed. That is, since the examiner can input both the fine movement instruction and the rough movement instruction by the tilt operation of tilting the operation stick, it is not required to switch a part to be operated between the fine movement and the rough movement. In addition, the examiner can switch between the fine movement instruction and the rough movement instruction by adjusting only the tilting angle of the operation stick without performing any operation of the operation stick other than the tilt operation (for example, sliding operation of the operation stick itself). In addition, since it is also possible to input the rough movement instruction by the rough movement operation unit, it is not required to significantly tilt the operation stick when inputting the rough movement instruction. Therefore, according to the ophthalmic apparatus of the present disclosure, both the fine movement instruction and the rough movement instruction for the optometry unit are input more easily and appropriately.

Techniques exemplified in the present disclosure can be applied to various ophthalmic apparatuses for performing examinations of a subject eye (for example, capturing an image of the subject eye, measurement of ocular characteristics of the subject eye, observation of the subject eye (including observation for surgery or treatment), and the like). Examples of the ophthalmic apparatus that captures an image of the subject eye include an OCT apparatus, a laser scanning optometry apparatus (SLO), a fundus camera, a goniometric apparatus, a corneal endothelial cell imaging apparatus (CEM), and the like. The ophthalmic apparatus that measure the ocular characteristics of the subject eye include an eye refractive power measurement apparatus, a corneal shape measurement apparatus, an eye axial length measurement apparatus, an intraocular pressure measurement apparatus, and the like. In addition, the technique exemplified in the present disclosure may be adopted in an ophthalmic apparatus such as a photocoagulator, a YAG laser surgical device, a slit lamp, or the like for performing surgery or treatment on a tissue of the subject eye while observing the subject eye.

A specific control method for finely moving the optometry unit can be selected as appropriate. For example, the control unit may finely move the optometry unit in the same direction as the tilt direction of the operation stick so that the tilting angle of the operation stick is proportional to the moving distance of the optometry unit. The control unit may finely move the optometry unit in response to the tilt operation of tilting the operation stick so that the tilted position of the operation stick and the position of the optometry unit correspond to each other.

In addition, a specific control method for roughly moving the optometry unit can also be selected as appropriate. For example, in a case where it is detected that the rough movement operation unit is operated, the control unit may roughly move the optometry unit in a direction corresponding to the direction where the rough movement operation unit is operated. In addition, in a case where a tilt operation of tilting the operation stick over a predetermined range is detected, the control unit may roughly move the optometry unit in a direction corresponding to the direction where the operation stick is tilted.

The control unit may set different speeds for the rough movement speed of the optometry unit in a case where a tilt operation of tilting the operation stick over a predetermined range is detected, and the rough movement speed of the optometry unit in a case where an operation of the rough movement operation unit is detected. In this case, the relative position of the optometry unit with respect to the subject eye can be likely to be more appropriately adjusted. For example, the control unit may set the rough movement speed of the optometry unit in a case where the tilt operation of tilting the operation stick over a predetermined range is detected to be slower than the rough movement speed of the optometry unit in a case where the operation of the rough movement operation unit is detected. In this case, discomfort is reduced when switching from a state where the operation stick is operated and the optometry unit is finely moved to a state where the tilt angle of the operation stick exceeds a predetermined range and the optometry unit is roughly moved.

The rough movement operation unit may be an annular member disposed on an outer periphery of the operation stick formed substantially in a rod shape to surround the operation stick. The rough movement operation unit may be supported so as to be slidable in two-dimensional directions. In this case, the examiner can input a rough movement instruction for the optometry unit in an appropriate direction by sliding the rough movement operation unit around the operation stick in the same direction as the tilt direction of the operation stick in a case where the optometry unit is moved, without performing the tilt operation of tilting the operation stick over the predetermined range. Therefore, the feeling of operation when the examiner operates the operation stick and the rough movement operation unit is improved. The rough movement operation unit may be supported so as to be slidable in two-dimensional directions independently of the operation stick. In this case, the examiner can easily input the rough movement instruction for the optometry unit by only sliding the rough movement operation unit independently.

The operation stick may be tilted independently of the rough movement operation unit in a case where the tilt angle is within a predetermined range. In a case where the tilt angle exceeds a predetermined range, the operation stick may be tilted while contacting the rough movement operation unit to operate the rough movement operation unit. In this case, when the tilt angle of the operation stick is within a predetermined range, the rough movement operation unit is not operated (for example, slid) even when the operation stick is tilted, so that the fine movement instruction for the optometry unit is appropriately input. In addition, when the tilt angle of the operation stick exceeds a predetermined range, the operation stick contacts the rough movement operation unit, and the rough movement operation unit is operated in the same direction as the tilt direction of the operation stick, so that a rough movement instruction for the optometry unit is input. Therefore, since the tilt direction of the operation stick when inputting a fine movement instruction coincides with the tilt direction of the operation stick when inputting a rough movement instruction, the feeling of operation is improved when the examiner inputs fine movement and rough movement instructions. Furthermore, the examiner can appropriately obtain whether or not a rough movement instruction is input to the ophthalmic apparatus depending on whether or not the tilted operation stick brings into contact with the rough movement operation unit to operate the rough movement operation unit. Therefore, both the fine movement instruction and the rough movement instruction for the optometry unit can be input more easily and appropriately.

In a case where the tilt angle exceeds a predetermined range, the operation stick may be tilted while contacting the annular rough movement operation unit to slide the rough movement operation unit. In this case, in the case of inputting the rough movement instruction for the optometry unit using the operation stick, the examiner can perform the tilt operation of tilting the operation stick in any direction on the two-dimensional plane in the same manner as in the case of inputting a fine movement instruction. However, the direction where the fine movement instruction can be input by the operation stick may be limited to some extent. For example, the operation stick may be configured to bring into contact with the rough movement operation unit to operate the rough movement operation unit only in a case where the tilt angle in a predetermined one-dimensional direction (for example, left-right direction) exceeds a predetermined range.

The operation detection unit may be provided with a rough movement operation detection unit detecting that the rough movement operation unit is operated. Both the tilt operation of tilting the operation stick over a predetermined range and the operation of the rough movement operation unit may be detected by the rough movement operation detection unit. In this case, even when the ophthalmic apparatus does not have a separate configuration for detecting that the tilt operation of tilting the operation stick over a predetermined range is performed, the ophthalmic apparatus can appropriately accept both a rough movement instruction by the tilt operation of tilting the operation stick and a rough movement instruction by the operation of the rough movement operation unit. Therefore, the optometry unit can be roughly moved appropriately with a simple configuration.

The ophthalmic apparatus may be further provided with a biasing unit. The biasing unit may bias the rough movement operation unit toward the initial position where the rough movement operation unit is in contact with the operation stick in a state where the tilt angle reaches the boundary of the predetermined range. When the tilt operation of tilting the operation stick over the predetermined range is completed, the biasing unit may move the rough movement operation unit toward the initial position to return the tilt angle of the operation stick being in contact with the rough movement operation unit within the predetermined range. In this case, when the rough movement instruction by the operation stick is completed and the examiner weakens the gripping force on the operation stick, not only the rough movement operation unit automatically returns to the initial position, but also the tilt angle of the operation stick automatically returns within the predetermined range for inputting the fine movement instruction. Therefore, the operability of the operation stick and the rough movement operation unit is further improved.

The operation detection unit may detect that the rough movement operation unit is operated in a case where the operation amount of the rough movement operation unit exceeds a specified amount. That is, a dead zone of operation may be provided from when the operation of the rough movement operation unit is started until the operation of the rough movement operation unit is actually detected. In this case, even in a case where the rough movement operation unit is erroneously slightly operated, the problem of unintended rough movement of the optometry unit is unlikely to occur. Therefore, operability is further improved.

It is also possible to change the configuration of at least one of the operation stick and the rough movement operation unit. For example, in a case where the tilt angle of the operation stick exceeds a predetermined range, the operation stick and the rough movement operation unit may not be in contact with each other. In this case, the ophthalmic apparatus may perform rough movement control of the optometry unit by detecting a tilt operation of tilting the operation stick over a predetermined range and an operation of the rough movement operation unit. Instead of a slidable annular member disposed to surround the outer periphery of the operation stick, a slidable member having another shape (for example, a non-annular shape such as a square and a circle), a knob, a trackball, or the like may be used as the rough movement operation unit.

In order to allow the examiner to appropriately perform various operations of the ophthalmic apparatus, it is important to be able to smoothly guide the movement of the operation member operated by the examiner. On the other hand, in the ophthalmic apparatus, the space for installing a guide unit for guiding the movement of the operation member is often limited. Therefore, the guide unit that can be easily installed in a narrow space and that can smoothly guide the movement of the operation member is desired.

In the present disclosure, one direction on a slide plane, which is a two-dimensional plane on which the rough movement operation unit is slid, is defined as an X direction. The direction intersecting the X direction on the slide plane is defined as a Z direction. The direction intersecting the slide plane is defined as a Y direction. The ophthalmic apparatus may further include a guide unit that guides the movement of the rough movement operation unit on the slide plane (that is, an XZ plane). The guide unit may include an X-direction linear guide unit, a Z-direction linear guide unit, an X-direction slide unit, and a Z-direction slide unit. The X-direction linear guide unit extends linearly in the X direction. The Z-direction linear guide unit extends linearly in the Z direction. The X-direction slide unit is attached to the X-direction linear guide unit and relatively slides in the X direction with respect to the attached X-direction linear guide unit. The Z-direction slide unit is attached to the Z-direction linear guide unit and relatively slides in the Z direction with respect to the attached Z-direction linear guide unit. The X-direction linear guide unit may slide in the X direction with respect to the X-direction slide unit to guide the movement of the rough movement operation unit in the X direction. The Z-direction slide unit may slide in the Z direction with respect to the Z-direction linear guide unit to guide the movement of the rough movement operation unit in the Z direction.

In a general guide unit used in an ophthalmic apparatus in the related art, it was necessary to separately provide a portion that guides the operation member in the X direction and a portion that guides the operation member in the Z direction at different positions in the Y direction. As a result, in particular, the size of the guide unit in the Y direction increased. On the other hand, as illustrated in the present disclosure, by adopting a configuration in which a slide unit is attached to each of a plurality of linear guide units in the guide unit, it is easy to reduce the size of the guide unit in the Y direction.

Furthermore, in the guide unit of the present disclosure, the X-direction linear guide unit slides with respect to the X-direction slide unit, while the Z-direction slide unit slides with respect to the Z-direction linear guide unit. As a result, while adopting the same configuration (configuration using a linear guide unit and a slide unit) in each of the X direction and the Z direction, in addition to the movement of the rough movement operation unit in the X direction and the Z direction, a movement in an oblique direction inclined to both the X and Z directions is also appropriately guided. Therefore, the guide unit of the present disclosure can be easily installed in a narrow space, and can smoothly guide the movement of the rough movement operation unit.

The specific configurations of the plurality of linear guide units and the plurality of slide units can be appropriately selected. For example, a shaft may be adopted for the linear guide unit. In this case, a bearing through which the shaft is inserted may be adopted in the slide unit. By adopting the shaft and the bearing, deviation or the like in the Y direction between both the shaft and the bearing is unlikely to occur. In addition, a guide rail may be adopted for the linear guide unit. In this case, a carriage or the like that moves relatively along the guide rail may be adopted for the slide unit.

In addition, in the embodiments described below, a case where the left-right direction of the ophthalmic apparatus is defined as the X direction, the front-rear direction of the ophthalmic apparatus is defined as the Z direction, and the vertical direction of the ophthalmic apparatus is defined as the Y direction will be exemplified. However, it goes without saying that each direction of XYZ can be appropriately set. For example, even in a case where the front-rear direction of the ophthalmic apparatus is defined as the X direction and the left-right direction of the ophthalmic apparatus is defined as the Z direction, the same effects can be obtained.

A pair of the X-direction linear guide units may be disposed on each of the +Z direction side and the -Z direction side from a central portion in an XZ direction of the rough movement operation unit, and may extend linearly in the X direction in parallel to each other. A pair of the Z-direction linear guide units may be disposed on each of the +X direction side and the -X direction side from the central portion in the XZ direction of the rough movement operation unit, and may extend linearly in the Z direction in parallel to each other. A pair of the X-direction slide units may be attached to each of the pair of X-direction linear guide units. A pair of the Z-direction slide units may be attached to each of the pair of Z-direction linear guide units.

In this case, the four linear guide units and the four slide units are disposed in each of the +X direction, -X direction, +Z direction, and -Z direction from the central portion so as to surround the central portion in the XZ direction of the rough movement operation unit, which is an example of an operation member. Therefore, the load applied from the rough movement operation unit to the guide unit can be easily distributed, as compared to the case where the guide unit is disposed closer to either direction with respect to the central portion of the rough movement operation unit. Furthermore, while adopting the same configuration (configuration using a linear guide unit and a slide unit) in each of the +X direction, -X direction, +Z direction, and -Z direction, in addition to the movement of the rough movement operation unit in the X direction and the Z direction, a movement in an oblique direction inclined to both the X and Z directions is also appropriately guided. Therefore, the movement of the rough movement operation unit on the XZ plane is easily guided more smoothly.

In addition, the four linear guide units may be disposed at positions that are four-fold rotationally symmetrical about the center of the rough movement operation unit in the XZ direction. In this case, the load applied from the rough movement operation unit to the guide unit is easily distributed more evenly. Therefore, the movement of the rough movement operation unit on the XZ plane is easily guided more smoothly.

However, it is also possible to set the number of each of the X-direction linear guide unit and the X-direction slide unit to one. In addition, it is also possible to set the number of each of the Z-direction linear guide unit and the Z-direction slide unit to one. Even in these cases, the guide unit of the present disclosure can be easily installed in a narrow space and can smoothly guide the movement of the rough movement operation unit.

The X-direction linear guide unit may be provided with an X-direction biasing unit that biases the position of the X-direction linear guide unit with respect to the X-direction slide unit toward the initial X position where the rough movement operation unit is not operated in the X direction. The Z-direction slide unit may be provided with a Z-direction biasing unit that biases the position of the Z-direction slide unit with respect to the Z-direction linear guide unit toward the initial Z position where the rough movement operation unit is not operated in the Z direction. In this case, the X-direction biasing unit and the Z-direction biasing unit bias the rough movement operation unit toward the initial position. As a result, it is easier for the rough movement operation unit to smoothly return to the initial position in the XZ direction.

As described above, the pair of X-direction linear guide units, the pair of Z-direction linear guide units, the pair of X-direction slide units, and the pair of Z-direction slide units may be used. Each of the pair of X-direction linear guide units may be provided with the X-direction biasing unit. In addition, each of the pair of Z-direction linear guide units may be provided with the Z-direction biasing unit. In this case, at each position in the +X direction, -X direction, +Z direction, and -Z direction with reference to the central portion in the XZ direction of the rough movement operation unit (an example of the operation member), the rough movement operation unit is biased toward the initial position. As a result, since the position in the XZ direction of the centers of gravity of the plurality of biasing units is closer to the center position in the XZ direction of the rough movement operation unit, the rough movement operation unit can be likely to smoothly return to the initial position in the XZ direction.

The X-direction linear guide unit and the Z-direction linear guide unit may be disposed on the same XZ plane. For example, in the case of using the pair of X-direction linear guide units and the pair of Z-direction linear guide units, the pair of X-direction linear guide units and the pair of Z-direction linear guide units (that is, four linear guide units) may be disposed on the same XZ plane. In this case, it is easy to reduce the size of the guide unit in the Y direction.

However, at least one of the plurality of linear guide units may be disposed at a position different from the position in the Y direction of the other linear guide units. Even in this case, by adopting a configuration in which a slide unit is attached to each of the plurality of linear guide units, it is easy to reduce the size of the guide unit.

When the rough movement operation unit is connected with the Z-direction slide unit, the Z-direction slide unit may also move on the XZ plane in synchronization with the movement of the rough movement operation unit on the XZ plane. When the X-direction linear guide unit and the Z-direction linear guide unit are connected with each other, the plurality of linear guide units may integrally move in the XZ direction. In a case where the pair of X-direction linear guide units and the pair of Z-direction linear guide units are used, by connecting the rough movement operation unit to each of the pair of Z-direction slide units, the pair of Z-direction slide units may also move on the XZ plane in synchronization with the movement of the rough movement operation unit on the XZ plane. When the pair of X-direction linear guide units and the pair of Z-direction linear guide units are connected with each other, the four linear guide units may integrally move in the XZ direction.

In this case, when the rough movement operation unit is moved in the Z direction, the Z-direction slide unit is also moved in the Z direction. As a result, since the Z-direction slide unit slides in the Z direction with respect to the Z-direction linear guide unit, the movement of the rough movement operation unit in the Z direction is appropriately guided. In addition, when the rough movement operation unit is moved in the X direction, since the Z-direction slide unit also moves in the X direction, the plurality of linear guide units connected with each other also move in the X direction together with the Z-direction slide unit. When the plurality of linear guide units integrally move in the X direction, since the X-direction linear guide unit moves in the X direction with respect to the X-direction slide unit, the movement of the rough movement operation unit in the X direction is also appropriately guided. Therefore, the movement of the rough movement operation unit on the XZ plane is appropriately guided. In a case where the X-direction linear guide unit and the Z-direction linear guide unit are connected with each other, the X-direction slide unit is fixed to a base unit which is a base.

However, instead of connecting the plurality of linear guide units, it is also possible to connect the plurality of slide units (an X-direction slide unit and a Z-direction slide unit). In the case of connecting the plurality of slide units, the rough movement operation unit is connected with the X-direction linear guide unit, so that the X-direction linear guide unit may also move on the XZ plane in synchronization with the movement of the rough movement operation unit on the XZ plane. In the case of using the pair of X-direction linear guide units, the pair of Z-direction linear guide units, the pair of X-direction slide units, and the pair of Z-direction slide units, four slide units (the pair of X-direction slide units and the pair of Z-direction slide units) may be connected. In the case of connecting the four slide units, the rough movement operation unit is connected with the pair of X-direction linear guide units, so that the pair of X-direction linear guide units may also move on the XZ plane in synchronization with the movement of the rough movement operation unit on the XZ plane.

In this case, when the rough movement operation unit is moved in the X direction, the X-direction linear guide unit is also moved in the X direction. As a result, since the X-direction linear guide unit slides in the X direction with respect to the X-direction slide unit, the movement of the rough movement operation unit in the X direction is appropriately guided. In addition, when the rough movement operation unit is moved in the Z direction, the X-direction linear guide unit also moves in the Z direction, so that the plurality of slide units connected with each other also move in the Z direction together with the X-direction linear guide unit. When the plurality of slide units integrally move in the Z direction, since the Z-direction slide unit moves in the Z direction with respect to the Z-direction linear guide unit, the movement of the rough movement operation unit in the Z direction is also appropriately guided. Therefore, the movement of the rough movement operation unit on the XZ plane is appropriately guided. In a case where the X-direction slide unit and the Z-direction slide unit are connected with each other, the Z-direction linear guide unit is fixed to the base unit which is a base.

The operation unit exemplified in the present disclosure is provided with an operation stick supported so as to be tiltable in any direction, a rough movement operation unit operated by the examiner for roughly moving the optometry unit, and an operation detection unit that detects the operation of tilting the operation stick and the rough movement operation unit. In a case where the operation detection unit detects a tilt operation of tilting the operation stick within a predetermined range, the position of the optometry unit is finely changed in response to the detected tilt operation. In a case where the operation detection unit detects at least one of the tilt operation of the operation stick over a predetermined range and the operation of the rough movement operation unit, the position of the optometry unit is roughly changed in response to the detected operation.

According to the operation unit in the present disclosure, the examiner can input a rough movement instruction for the optometry unit even when either a tilt operation of tilting the operation stick over a predetermined range or an operation of the rough movement operation unit is performed. That is, since the examiner can input both the fine movement instruction and the rough movement instruction by the tilt operation of tilting the operation stick, it is not required to switch a part to be operated between the fine movement and the rough movement. In addition, the examiner can switch between the fine movement instruction and the rough movement instruction by adjusting only the tilt angle of the operation stick without performing any operation other than the tilt operation of the operation stick (for example, sliding operation of the operation stick itself). In addition, since it is also possible to input the rough movement instruction by the rough movement operation unit, it is not required to significantly tilt the operation stick when inputting the rough movement instruction. Therefore, both the fine movement instruction and the rough movement instruction for the optometry unit can be input more easily and appropriately.

In addition, the operation unit exemplified in the present disclosure can also be expressed as follows.

An operation unit operated by an examiner to change a relative position of an optometry unit of an ophthalmic apparatus with respect to a subject eye, the unit including an operation member that is moved on a slide plane, which is a two-dimensional plane, by being operated by the examiner to move the optometry unit, and a guide unit that guides a movement of the operation member on the slide plane, in which in a case where one direction on the slide plane is defined as the X direction, the direction that intersects the X direction on the slide plane is defined as the Z direction, and the direction that intersects the slide plane is defined as the Y direction, the guide unit has an X-direction linear guide unit linearly extending in the X direction, a Z-direction linear guide unit linearly extending in the Z direction, an X-direction slide unit attached to the X-direction linear guide unit and relatively sliding in the X direction with respect to the X-direction linear guide unit to which the X-direction slide unit is attached, and a Z-direction slide unit attached to the Z-direction linear guide unit and relatively sliding in the Z direction with respect to the Z-direction linear guide unit to which the Z-direction slide unit is attached, the X-direction linear guide unit slides in the X direction with respect to the X-direction slide unit to guide a movement of the rough movement operation unit in the X direction, and the Z-direction slide unit slides in the Z direction with respect to the Z-direction linear guide unit to guide a movement of the rough movement operation unit in the Z direction.

### <Embodiment>

Hereinafter, one of the typical embodiments according to the present disclosure will be described with reference to the drawings. The ophthalmic apparatus 1 of the present embodiment examines a subject eye E in a state where the relative position with respect to the subject eye E is adjusted to an appropriate position (for example, the examination axis is aligned with the subject eye E). The ophthalmic apparatus 1 exemplified in the present embodiment is an eye refractive power measurement apparatus that measures the eye refractive power of the subject eye E. However, the ophthalmic apparatus 1 may be an apparatus that performs an examination different from eye refractive power measurement (for example, an OCT apparatus, a laser scanning optometry apparatus (SLO), a fundus camera, a goniometric apparatus, a corneal endothelial cell imaging apparatus (CEM), a corneal curvature measurement apparatus, an intraocular pressure measurement apparatus, an eye axial length measurement apparatus, and the like). In the following description, the optical axis direction of the light used for examination is defined as the Z-axis direction (front-rear direction), the horizontal direction perpendicular to the Z-axis direction is defined as the X-axis direction (left-right direction), and the direction perpendicular to both the Z-axis and the X-axis is defined as the Y-axis direction (vertical direction).

### (Schematic Configuration)

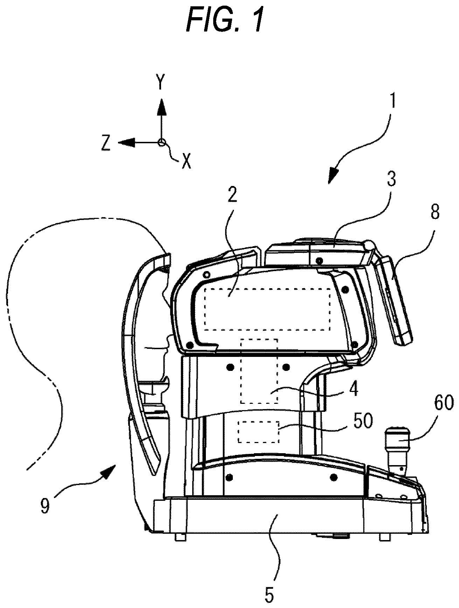

A schematic configuration of the ophthalmic apparatus 1 will be described with reference to FIG. 1. The ophthalmic apparatus 1 of the present embodiment is provided with a housing 3, a base 5, a face support unit 9, an optometry unit 2, an operation unit 60, a drive unit 4, a control unit 50, a display unit 8, and the like. The housing 3 is provided with a base 5 and various components of the ophthalmic apparatus 1 (for example, the optometry unit 2, the drive unit 4, the control unit 50, and the like). The base 5 of the housing 3 supports the entire ophthalmic apparatus 1. The face support unit 9 supports the face of a subject. The face support unit 9 of the present embodiment is provided with a chin rest on which the jaw of the subject is placed and the forehead rest on which a forehead rest of the subject is applied. Although the face support unit 9 in the present embodiment is provided on the base 5, the face support unit 9 may be provided independently of the base 5.

The optometry unit 2 examines the subject eye E. The optometry unit 2 may be provided with, for example, a configuration (optical system in the present embodiment) for examining at least one of the eye refractive power, corneal curvature, intraocular pressure, and the like of the subject eye E. In addition, the optometry unit 2 may be provided with an optical system for capturing an image of the tissue of the subject eye. The drive unit 4 includes an actuator which moves the optometry unit 2 in the up, down, left, right, front, and rear directions (three-dimensional directions) with respect to the base 5 to change the relative position between the subject eye E and the optometry unit 2. The actuator of the drive unit 4 may move the face support unit 9 together with the optometry unit 2 or instead of the optometry unit 2 to change the relative position between the subject eye E and the optometry unit 2. The operation unit 60 is disposed on the side of the housing 3 opposite to the side where the subject is located (that is, the side where the examiner is located). The operation unit 60 is operated by the examiner for inputting an instruction to move the optometry unit 2, an instruction to start examination execution, and the like. Details of the operation unit 60 will be described later. The control unit 50 controls various manages in the ophthalmic apparatus 1 (for example, drive control of the drive unit 4, and the like). The display unit 8 displays various images (for example, an observation image of the subject eye E, measurement results, and the like). In the present embodiment, a touch panel is provided on the surface of the display unit 8. The touch panel is used as one of the operation units operated by an examiner for inputting various instructions. The display unit 8 may be provided independently of the housing 3.

### (Optometry Unit and Control Unit)

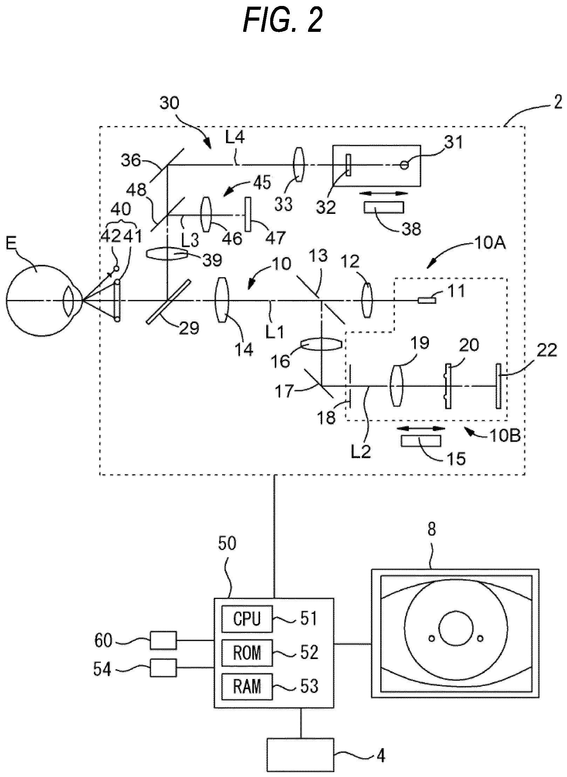

The optometry unit 2 and the control unit 50 will be described with reference to FIG. 2. As described above, in the present embodiment, the case where the ophthalmic apparatus 1 is an eye refractive power measurement apparatus is exemplified. Therefore, the optometry unit 2 of the present embodiment is provided with an optical system for measuring the eye refractive power of the subject eye. Specifically, the optometry unit 2 of the present embodiment is provided with a measuring optical system 10, a fixation target presenting optical system 30, an index projecting optical system 40, and an observation optical system (imaging optical system) 45.

The measuring optical system 10 is provided with a projecting optical system (light projecting optical system) 10A and a light receiving optical system 10B. The projecting optical system 10A projects a light flux onto the fundus of the subject eye E through the pupil of the subject eye E. The light receiving optical system 10B extracts a ring-shaped reflected light flux from the fundus via the peripheral portion of the pupil, and captures an image of a ring-shaped fundus reflected image mainly used for refractive power measurement.

The projecting optical system 10A is provided with a measurement light source 11, a relay lens 12, a hole mirror 13, and an objective lens 14 on an optical axis L1. The measurement light source 11 projects a spot light source image onto the fundus through the optical members from the relay lens 12 to the objective lens 14 and the center of the pupil of the subject eye E. The measurement light source 11 is moved in the optical axis L1 direction by a moving mechanism 15. The hole mirror 13 is provided with an aperture through which the light flux from the measurement light source 11 via the relay lens 12 passes. The hole mirror 13 is disposed at a position optically conjugate with the pupil of the subject eye E.

The light receiving optical system 10B shares the hole mirror 13 and the objective lens 14 with the projecting optical system 10A. In addition, the light receiving optical system 10B is provided with a relay lens 16, a total reflection mirror 17, a light receiving diaphragm 18, a collimator lens 19, a ring lens 20, and an imaging element 22 on an optical axis L2 in the reflection direction of the hole mirror 13. The light receiving diaphragm 18, the collimator lens 19, the ring lens 20, and the imaging element 22 are moved in the direction of the optical axis L2 by the moving mechanism 15 together with the measurement light source 11 of the projecting optical system 10A. In a case where the measurement light source 11 is disposed at a position conjugated with the fundus by the moving mechanism 15, the light receiving diaphragm 18 and the imaging element 22 are also disposed at a position optically conjugated with the fundus.

The ring lens 20 is an optical element for shaping the fundus reflected light guided from the objective lens 14 via the collimator lens 19 into a ring shape. The ring lens 20 includes a ring-shaped lens unit and a light shielding unit. In a case where the light receiving diaphragm 18 and the imaging element 22 are disposed at positions optically conjugated with the fundus, the ring lens 20 is disposed at a position optically conjugated with the pupil of the subject eye E. The imaging element 22 receives ring-shaped fundus reflected light (hereinafter referred to as "ring image") via the ring lens 20. The imaging element 22 outputs image information on the received ring image to the control unit 50. As a result, the control unit 50 performs the display of the ring image on the display unit 8, the calculation of refractive power based on the ring image, and the like.

In the present embodiment, a dichroic mirror 29 is disposed between the objective lens 14 and the subject eye E. The dichroic mirror 29 transmits the light emitted from the measurement light source 11 and the fundus reflected light corresponding to the light from the measurement light source 11, while guiding the light flux from the fixation target presenting optical system 30 (details will be described later) to the subject eye. Furthermore, the dichroic mirror 29 reflects the anterior segment reflected light of the light from the index projecting optical system 40 (details will be described later) and guides the anterior segment reflected light to the observation optical system 45.

The index projecting optical system 40 is disposed in front of the subject eye E. The index projecting optical system 40 mainly projects an index used for alignment of the optical system with respect to the subject eye E onto an anterior segment of the subject eye E. In the present embodiment, the index projecting optical system 40 projects an index used for alignment of the optical system with respect to the subject eye E in at least one of the XY directions and the Z direction onto the anterior segment of the eye. The ophthalmic apparatus 1 can also perform alignment by detecting a characteristic portion in the anterior segment image without using the index projecting optical system 40.

The index projecting optical system 40 of the present embodiment is provided with a ring index projection unit 41 and an index projection unit 42. The ring index projection unit 41 projects diffused light onto the cornea of the subject eye E to project a ring index (so-called Mayer ring) onto the cornea. In the present embodiment, the ring index projection unit 41 is also used as an anterior segment illumination for illuminating the anterior segment of the subject eye E. The index projection unit 42 projects parallel light onto the cornea of the subject eye E to project an infinity index onto the cornea.

The fixation target presenting optical system 30 is provided with a light source 31, a fixation target 32, a relay lens 33, a reflection mirror 36, and a lens 39 on an optical axis L4. The fixation target 32 is used for fixating the subject eye E during objective refractive power measurement. For example, by illuminating the fixation target 32 with the light source 31, light for fixating the subject eye E is projected onto the subject eye E. The light source 31 and the fixation target 32 are integrally moved in the direction of the optical axis L4 by a driving mechanism 38. The movement of the light source 31 and the fixation target 32 changes the presentation position (presentation distance) of the fixation target. As a result, the subject eye E is fogged and the refractive power is measured.

The observation optical system 45 is provided with an imaging lens 46 and an imaging element 47 on an optical axis L3 in the reflection direction of a half mirror 48. The imaging element 47 is disposed at a position optically conjugate with the anterior segment of the subject eye E. The imaging element 47 captures an image of the anterior segment of the eye illuminated by the ring index projection unit 41. An output from the imaging element 47 is input to the control unit 50. As a result, the anterior segment image of the subject eye E captured by the imaging element 47 is displayed on the display unit 8 (refer to FIG. 2). In addition, the imaging element 47 captures an image of an alignment index image (a ring index and an infinity index in the present embodiment) formed on the cornea of the subject eye E by the index projecting optical system 40. As a result, the control unit 50 can detect the alignment index image based on the imaging result of the imaging element 47. The control unit 50 can determine whether or not the alignment state is appropriate based on the position at which the alignment index image is detected.

The control unit 50 controls various manages of the ophthalmic apparatus 1 (for example, drive control of the drive unit 4, and the like). The control unit 50 is provided with a CPU 51, a ROM 52, a RAM 53, and the like. The CPU 51 is a processor that manages control. The ROM 52 stores an ophthalmic apparatus control program for controlling the ophthalmic apparatus 1, initial values, and the like. The RAM 53 temporarily stores various information. The control unit 50 is connected with the optometry unit 2, the drive unit 4, the display unit 8, the operation unit 60, and a storage unit (for example, non-volatile memory) 54. The storage unit 54 is a non-transitory storage medium that can hold stored contents even when the power supply is cut off. For example, a hard disk drive, or a detachable USB memory can be used as the storage unit 54.

### (Operation Unit of First Embodiment)

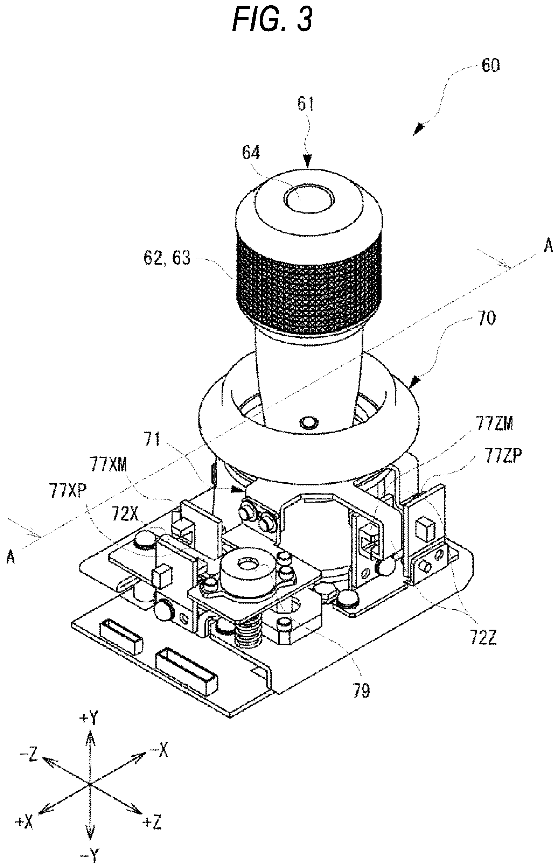

The operation unit 60 of the first embodiment will be described with reference to FIGS. 3 to 5. The operation unit 60 is operated by an examiner for inputting an instruction to move the optometry unit 2 of the ophthalmic apparatus 1 (in the present embodiment, an instruction to finely move and an instruction to roughly move the optometry unit 2) to the ophthalmic apparatus 1. When the operation unit 60 is operated, the control unit 50 outputs a signal corresponding to the content of the operation to the drive unit 4 to change the relative position of the optometry unit 2 with respect to the subject eye E.

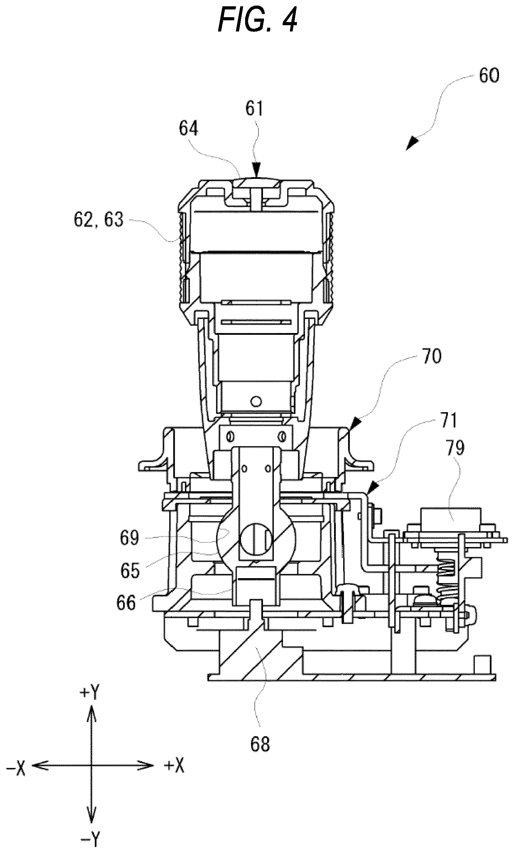

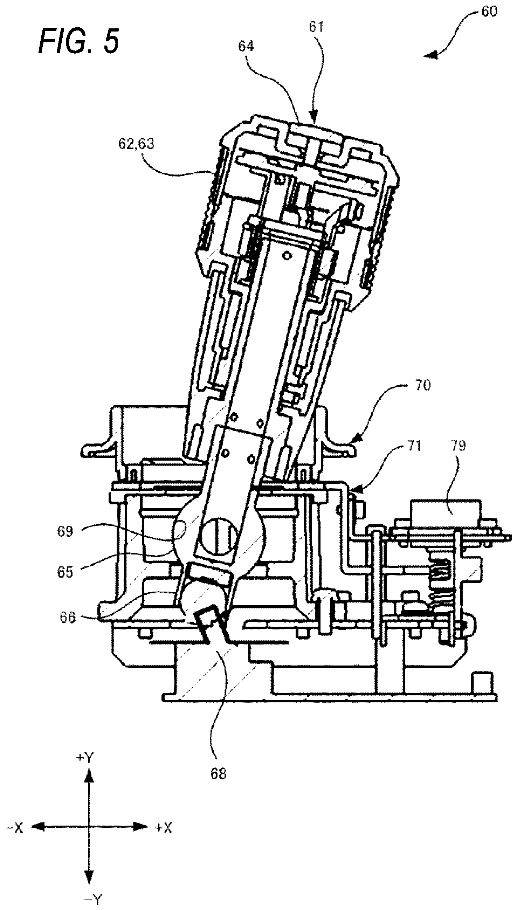

The operation unit 60 is provided on the housing 3 of the ophthalmic apparatus 1 (specifically, the base 5 of the housing 3, refer to FIG. 1). However, in order to facilitate understanding of the configuration, FIGS. 3 to 5 illustrate the operation unit 60 removed from the housing 3. The operation unit 60 may be provided independently of the housing 3 of the ophthalmic apparatus 1. In this case, the operation unit 60 may be connected with the ophthalmic apparatus 1 by wired communication or wireless communication. FIG. 3 is a perspective view of the operation unit 60 as viewed diagonally from the rear right. FIG. 4 is a cross-sectional view taken along the line A-A in FIG. 3. FIG. 5 is a view illustrating a state where the operation stick 61 is tilted in the +X direction over a predetermined range from the state illustrated in FIG. 3. In FIG. 3, the left front side of the paper is defined as +X direction, the right rear side of the paper is defined as -X direction, the upper side of the paper is defined as +Y direction, the lower side of the paper is defined as -Y direction, the right front side of the paper is defined as +Z direction, and the left rear side of the paper is defined as -Z direction. In FIGS. 4 and 5, the right side is defined as the +X direction, the left side is defined as the -X direction, the upper side is defined as the +Y direction, the lower side is defined as the -Y direction, the rear side of the paper is defined as the +Z direction, and the front side of the paper is defined as the -Z direction.

### (Schematic Configuration of Operation Unit of First Embodiment)

As illustrated in FIGS. 3 to 5, the operation unit 60 of the first embodiment is provided with an operation stick (sometimes referred to as a joystick) 61 and a rough movement operation unit 70. The operation stick 61 of the first embodiment extends outward (upward in the present embodiment) from the housing 3 (more specifically, the base 5 of the housing 3, refer to FIG. 1). The operation stick 61 is supported so as to be tiltable in any direction. The rough movement operation unit 70 is operated by the examiner for roughly moving the optometry unit 2. In addition, the operation unit 60 is provided with an operation detection unit that detects operations of the operation stick 61 and the rough movement operation unit 70. Although details will be described later, the operation detection unit of the first embodiment includes a detector having a tilt detection unit 68 (refer to FIGS. 4 and 5) and a rough movement operation detection unit 77 (77XP, 77XM, 77ZP, 77ZM, refer to FIG. 3).

In the present embodiment, when a tilt operation of tilting the operation stick 61 within a predetermined range is detected, the position of the optometry unit 2 is finely changed in response to the detected tilt operation (for example, according to the tilt direction and the amount of tilt (tilt angle) of the operation stick 61). When a tilt operation of tilting the operation stick 61 over a predetermined range is detected, the position of the optometry unit 2 is roughly changed in response to the detected tilt operation (according to the tilt direction of the operation stick 61). In addition, when the operation of the rough movement operation unit 70 is detected, the position of the optometry unit 2 is roughly changed in response to the detected operation (according to the operation direction of the rough movement operation unit 70). That is, in the ophthalmic apparatus 1 of the present embodiment, the examiner can input a rough movement instruction for the optometry unit 2 even when either a tilt operation of tilting the operation stick 61 over a predetermined range or an operation of the rough movement operation unit 70 is performed. That is, since the examiner can input both fine movement instruction and the rough movement instruction by the tilt operation of tilting the operation stick 61, it is not required to switch a part to be operated between fine movement and rough movement. In addition, the examiner can switch between the fine movement instruction and the rough movement instruction by adjusting only the tilt angle of the operation stick 61 without performing any operation of the operation stick 61 other than the tilt operation (for example, sliding operation of the operation stick 61 itself). In addition, since it is possible to input the rough movement instruction by the rough movement operation unit 70, it is not required to significantly tilt the operation stick 61 when inputting the rough movement instruction. Therefore, both the fine movement instruction and the rough movement instruction of the optometry unit 2 can be input more easily and appropriately. Hereinafter, each configuration will be described in more detail.

### (Operation Stick of First Embodiment)

As illustrated in FIGS. 3 to 5, the operation stick 61 is a substantially rod-shaped member that is gripped by the examiner. The operation stick 61 of the first embodiment extends upward from the housing 3. The operation stick 61 of the first embodiment can be tilted in any direction within a predetermined tilting range. When the tilt angle of the operation stick 61 reaches the limit of the tilting range, the operation stick 61 can no longer be tilted. The predetermined range in which the optometry unit 2 is finely moved is located on the center side of the tilting range of the operation stick 61. Within the tilting range, the outside of the predetermined range in which the optometry unit 2 is finely moved serves as an area for inputting a rough movement instruction. By tilting the operation stick 61 within a predetermined range in a direction where the optometry unit 2 is desired to move, the examiner can input an operation instruction for finely moving the optometry unit 2 in the direction corresponding to the tilt direction of the operation stick 61 among the XZ directions. In addition, by tilting the operation stick 61 over a predetermined range in the direction where the optometry unit 2 is desired to move, the examiner can input an operation instruction for roughly moving the optometry unit 2 in the direction corresponding to the tilt direction of the operation stick 61 among the XZ directions.

The operation stick 61 is provided with a grip unit 62, a rotary dial 63, a measurement button 64, a ball unit 65 (refer to FIGS. 4 and 5), and an action unit 66 (refer to FIGS. 4 and 5). The grip unit 62 is gripped by the examiner. The rotary dial 63 is operated by the examiner for inputting an instruction to the ophthalmic apparatus 1 to move the optometry unit 2 in the vertical direction (Y direction). The rotary dial 63 of the first embodiment is provided on the side surface of the grip unit 62 of the operation stick 61 along the circumferential direction. Rotation of the rotary dial 63 is detected by a rotation detection unit (for example, an encoder or the like). For example, when the rotary dial 63 is rotated clockwise, an operation signal for moving the optometry unit 2 upward (+Y direction) is output, and when the rotary dial 63 is rotated counterclockwise, an operation signal for moving the optometry unit 2 downward (-Y direction) is output. The measurement button 64 is operated by the examiner for inputting an instruction to start the examination of the subject eye E by the optometry unit 2. The measurement button 64 of the first embodiment is provided above the operation stick 61.

As illustrated in FIGS. 4 and 5, the ball unit 65 is positioned closer to the proximal side (lower in the first embodiment) than the grip unit 62 of the substantially rod-shaped operation stick 61. The ball unit 65 is rotatably held by a bearing unit 69. As a result, the operation stick 61 is tilted in any direction with the ball unit 65 as the center. The action unit 66 is positioned closer to the proximal side than the ball unit 65 (that is, on the side opposite to the grip unit 62 with the ball unit 65 interposed therebetween) of the substantially rod-shaped operation stick 61. Therefore, when the operation stick 61 is tilted, the action unit 66 moves in the direction opposite to the tilt direction of the grip unit 62 of the operation stick 61. With the above configuration, the configuration for detecting the tilt operation of tilting the operation stick 61 is likely to be accommodated appropriately inside the housing 3 (refer to FIG. 1).

The tilt detection unit 68 is provided on a part of the operation stick 61 or a member that moves in conjunction with the tilt operation of tilting the operation stick 61. The tilt detection unit 68 of the first embodiment is connected with the action unit 66 of the operation stick 61. The tilt detection unit 68 detects the operation direction and the operation amount (tilt angle) of the tilt operation of tilting the operation stick 61. In the first embodiment, the detection result of the tilt operation of tilting the operation stick 61 by the tilt detection unit 68 is used when the optometry unit 2 is finely moved. It is also possible to change the configuration for detecting the tilt operation of tilting the operation stick 61.

### (Rough Movement Operation Unit of First Embodiment)

As illustrated in FIGS. 3 to 5, the rough movement operation unit 70 of the first embodiment is an annular (substantially toric in the present embodiment) member disposed to surround the outer periphery of the operation stick 61 formed substantially in a rod shape. The rough movement operation unit 70 is supported so as to be slidable in two-dimensional directions on the XZ plane. Therefore, the examiner can input the rough movement instruction for the optometry unit 2 in an appropriate direction by sliding the rough movement operation unit 70 around the operation stick 61 in the same direction as the tilt direction of the operation stick 61 in a case where the optometry unit 2 is moved, without performing the tilt operation of tilting the operation stick 61 over the predetermined range. Therefore, the feeling of operation when the examiner operates the operation stick 61 and the rough movement operation unit 70 is improved.

A rough movement operation interlocking unit 71 is connected with the rough movement operation unit 70. The rough movement operation interlocking unit 71 moves in conjunction with the operation of the rough movement operation unit 70 (sliding operation on the XZ plane in the first embodiment). As an example, the rough movement operation interlocking unit 71 of the first embodiment is configured by bending and connecting a substantially plate-like member. When the rough movement operation unit 70 slides on the XZ plane, the rough movement operation interlocking unit 71 also slides on the XZ plane.

As illustrated in FIG. 3, the rough movement operation interlocking unit 71 is provided with an X detected unit 72X and a Z detected unit 72Z. In the main body of the operation unit 60, a +X rough movement operation detection unit 77XP is provided on the +X side of the X detected unit 72X, and a -X rough movement operation detection unit 77XM is provided on the - X side. When the rough movement operation unit 70 is operated in the +X direction, the +X rough movement operation detection unit 77XP detects that the rough movement operation unit 70 is operated in the +X direction by detecting that the X detected unit 72X of the rough movement operation interlocking unit 71 moves in the +X direction. In addition, when the rough movement operation unit 70 is operated in the -X direction, the -X rough movement operation detection unit 77XM detects that the rough movement operation unit 70 is operated in the -X direction by detecting that the X detected unit 72X of the rough movement operation interlocking unit 71 moves in the -X direction.

In the main body of the operation unit 60, a +Z rough movement operation detection unit 77ZP is provided on the +Z side of the Z detected unit 72Z, and a -Z rough movement operation detection unit 77ZM is provided on the -Z side. When the rough movement operation unit 70 is operated in the +Z direction, the +Z rough movement operation detection unit 77ZP detects that the rough movement operation unit 70 is operated in the +Z direction by detecting that the Z detected unit 72Z of the rough movement operation interlocking unit 71 moves in the +Z direction. In addition, when the rough movement operation unit 70 is operated in the -Z direction, the -Z rough movement operation detection unit 77ZM detects that the rough movement operation unit 70 is operated in the -Z direction by detecting that the Z detected unit 72Z of the rough movement operation interlocking unit 71 moves in the -Z direction.

In the first embodiment, the term "detection of operation of the rough movement operation unit 70" includes not only the detection of direct operation of the rough movement operation unit 70 by the examiner, but also the detection of tilt operation of tilting the operation stick 61 over a predetermined range. The details will be described later. In addition, various detection elements (for example, photoelectric switches, and the like) can be used for the rough movement operation detection units 77 (77XP, 77XM, 77ZP, 77ZM).

Each of the plurality of rough movement operation detection units 77 (77XP, 77XM, 77ZP, 77ZM) detects that the rough movement operation unit 70 is operated in a case where the operation amount of the rough movement operation unit 70 exceeds a specified amount. As an example, the +X rough movement operation detection unit 77XP and the -X rough movement operation detection unit 77XM are disposed such that a certain distance is provided between a position where each of +X rough movement operation detection unit 77XP and -X rough movement operation detection unit 77XM can detect the X detected unit 72X and a position of the X detected unit 72X in the initial position where the rough movement operation unit 70 is not operated. Similarly, the +Z rough movement operation detection unit 77ZP and the -Z rough movement operation detection unit 77ZM are disposed such that a certain distance is provided between a position where each of +Z rough movement operation detection unit 77ZP and -Z rough movement operation detection unit 77ZM can detect the Z detected unit 72Z and a position of the Z detected unit 72Z in the initial position where the rough movement operation unit 70 is not operated. As a result, it is detected for the first time that the rough movement operation unit 70 is operated when the operation amount of the rough movement operation unit 70 exceeds the specified amount (that is, the distance between the detected unit and the position detectable by the rough movement operation detection unit 77 in a state where the rough movement operation unit 70 is at the initial position). That is, a dead zone of operation may be provided from when the operation of the rough movement operation unit 70 is started until the operation of the rough movement operation unit 70 is actually detected. In this case, even in a case where the rough movement operation unit 70 is erroneously slightly operated, the problem of unintended rough movement of the optometry unit 2 is unlikely to occur. Therefore, operability is further improved.

As illustrated in FIGS. 3 to 5, a biasing unit 79 is provided in a part of the rough movement operation interlocking unit 71. The biasing unit 79 biases the position of the rough movement operation unit 70 connected with the rough movement operation interlocking unit 71 toward the initial position. The initial position can also be expressed as a standby position of the rough movement operation unit 70 in a state where the rough movement operation unit 70 is not operated and the tilt operation of tilting the operation stick 61 is not performed exceeding a predetermined range.

### (Method of Inputting Rough Movement Instruction)

A method of inputting a rough movement instruction in the ophthalmic apparatus 1 of the present embodiment will be described. The method of inputting a rough movement instruction is common to the first embodiment and a second embodiment described later. As described above, in the present embodiment, the examiner can input an instruction to roughly move the optometry unit 2 in the direction where the rough movement operation unit 70 is moved by sliding the rough movement operation unit 70 in a desired direction. Furthermore, in the present embodiment, the examiner can input an instruction to roughly move the optometry unit 2 in the direction where the operation stick 61 is tilted by tilt the operation stick 61 over a predetermined range in a desired direction. Specifically, as illustrated in FIGS. 4 and 5, the operation stick 61 of the present embodiment is tilted independently of the rough movement operation unit 70 in a case where the tilt angle is within a predetermined range. In this case, when the tilt angle of the operation stick 61 is within a predetermined range, since the rough movement operation unit 70 is not slid even when the operation stick 61 is tilted, the fine movement instruction of the optometry unit 2 is appropriately input. On the other hand, when the tilt angle exceeds the predetermined range, the operation stick 61 comes into contact with the annular rough movement operation unit 70 (more specifically, comes into contact with the inner peripheral edge portion of the rough movement operation unit 70), and is tilted while sliding the rough movement operation unit 70. Therefore, since the tilt direction of the operation stick 61 when inputting a fine movement instruction coincides with the tilt direction of the operation stick 61 when inputting a rough movement instruction, the feeling of operation is improved when the examiner inputs fine movement and rough movement instructions. Furthermore, the examiner can appropriately obtain whether or not a rough movement instruction is input to the ophthalmic apparatus 1 depending on whether or not the tilted operation stick 61 brings into contact with the rough movement operation unit 70 to slide the rough movement operation unit 70. Therefore, both the fine movement instruction and the rough movement instruction of the optometry unit 2 can be input more easily and appropriately.

By detecting that the rough movement operation unit 70 is slid, the rough movement operation detection unit 77 (a part of the operation detection unit) of the present embodiment detects both the tilt operation of tilting the operation stick 61 over a predetermined range and the direct operation of the rough movement operation unit 70 by the examiner. Therefore, even when there is no configuration for detecting that the tilt operation of tilting the operation stick 61 over a predetermined range is performed, the ophthalmic apparatus 1 can appropriately accept both a rough movement instruction by the tilt operation of tilting the operation stick 61 and a rough movement instruction by the sliding operation of the rough movement operation unit 70.

As described above, the biasing unit 79 biases the position of the rough movement operation unit 70 connected with the rough movement operation interlocking unit 71 toward the initial position. In addition, when the tilt angle exceeds a predetermined range, the operation stick 61 comes into contact with the annular rough movement operation unit 70. Therefore, when the tilt operation of tilting the operation stick 61 over the predetermined range is completed, the biasing unit 79 can move the rough movement operation unit 70 to the initial position and return the tilt angle of the operation stick 61 in contact with the rough movement operation unit 70 within a predetermined range. That is, when the rough movement instruction by the operation stick 61 is completed and the examiner weakens the gripping force on the operation stick 61, not only the rough movement operation unit 70 automatically returns to the initial position, but also the tilt angle of the operation stick 61 automatically returns within the predetermined range for inputting the fine movement instruction. Therefore, the operability of the operation stick 61 and the rough movement operation unit 70 is further improved.

### (Relative Position Adjustment Processing)

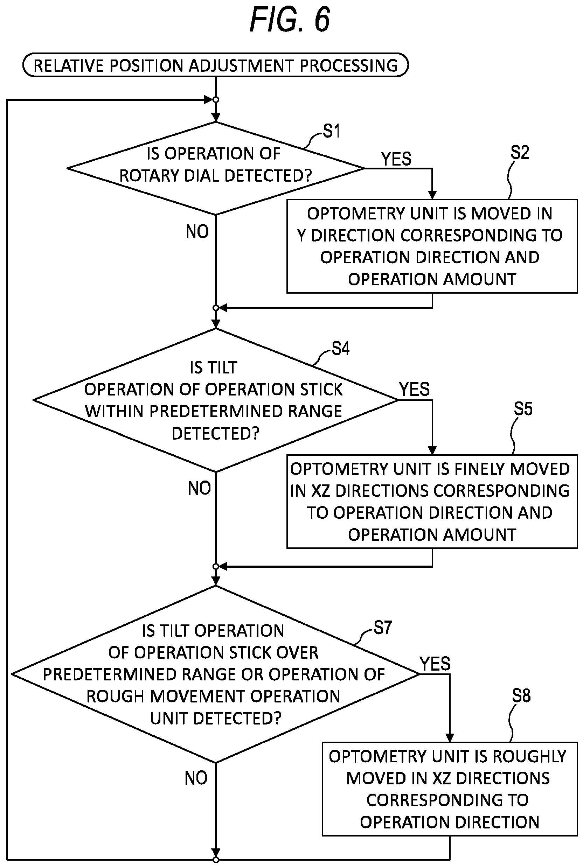

Relative position adjustment processing performed by the ophthalmic apparatus 1 of the present embodiment will be described with reference to FIG. 6. The relative position adjustment processing can be performed both in the first embodiment and in a second embodiment described later. In the relative position adjustment processing, the relative position between the subject eye E and the optometry unit 2 is adjusted by controlling the driving of the drive unit 4 according to the operation instruction input by the examiner and moving the position of the optometry unit 2. When the power is turned on, the control unit 50 (CPU 51) of the ophthalmic apparatus 1 performs the relative position adjustment processing illustrated in FIG. 6 according to the ophthalmic apparatus control program stored in a storage device (for example, the ROM 52 or the like).

First, the control unit 50 determines whether or not an operation of the rotary dial 63 is detected (S 1). When the operation of the rotary dial 63 is not detected (S 1: NO), the control unit 50 determines whether or not the tilt operation of tilting the operation stick 61 within a predetermined range is detected by the tilt detection unit 68 (S4). When the tilt operation of tilting the operation stick 61 within the predetermined range is not detected (S4: NO), the control unit 50 determines whether or not a tilt operation of tilting the operation stick 61 over a predetermined range or an operation of the rough movement operation unit 70 is detected (S7). When none of the operations is detected (S7: NO), the processing returns to S1, and the processing of S1 to S7 are repeated.

When the operation of the rotary dial 63 is detected (S1: YES), the control unit 50 controls driving of the drive unit 4 according to the detected operation direction (rotation direction) and operation amount (rotation amount) of the rotary dial 63, so that the position of the optometry unit 2 in the vertical direction (Y direction) is changed in the direction corresponding to the operation direction by a distance corresponding to the operation amount (S2). Thereafter, the processing proceeds to S4. The ophthalmic apparatus 1 may adjust the relative position between the subject eye E and the optometry unit 2 by moving the face support unit 9 together with the optometry unit 2 or separately from the optometry unit 2 according to the operation of the rotary dial 63 in the vertical direction.

When the tilt detection unit 68 detects the tilt operation of tilting the operation stick 61 within a predetermined range (S4: YES), the control unit 50 controls the driving of the drive unit 4 in response to the detected tilt direction and tilt amount of the operation stick 61, so that the position of the optometry unit 2 in the front, rear, left, and right directions (XZ directions) is finely shifted in the direction corresponding to the tilt direction by a distance corresponding to the tilt amount (S5). Thereafter, the processing proceeds to S7. As described above, the fine movement means moving the optometry unit 2 smaller (or slower) than during the rough movement in order to finely adjust the relative position between the subject eye E and the optometry unit 2. In the present embodiment, the control unit 50 controls the optometry unit 2 to move smaller than the rough movement, by allowing the optometry unit 2 to move within a movable range (within a movable range corresponding to a tiltable range of the operation stick 61) which is smaller than a movable range for the rough movement. A specific method of fine movement control in S5 can be changed as appropriate. For example, the control unit 50 may finely move the optometry unit 2 in a direction corresponding to the tilt direction of the operation stick 61 so that the tilt angle (tilt amount) of the operation stick 61 is proportional to the moving distance of the optometry unit 2. The control unit 50 may finely move the optometry unit 2 in response to the tilt operation of tilting the operation stick 61 so that the tilted position of the operation stick 61 and the position of the optometry unit 2 correspond to each other. In the present embodiment, a movement speed (for example, maximum movement speed) of the optometry unit 2 during the fine movement is lower than a movement speed of the optometry unit 2 during the rough movement. However, the movement speed (maximum movement speed) of the optometry unit 2 during the fine movement may be equal to the movement speed of the optometry unit 2 during the rough movement.

When a tilt operation of tilting the operation stick 61 over a predetermined range or an operation of the rough movement operation unit 70 is detected (S7: YES), the control unit 50 controls the driving of the drive unit 4 in response to the detected operation direction and operation amount of the operation stick 61 or the rough movement operation unit 70 (for example, the operation time of the switch), so that the position of the optometry unit 2 in the front, rear, left, and right directions (XZ directions) is roughly shifted in the direction corresponding to the operation direction (S8). Thereafter, the processing returns to S 1. As described above, the rough movement is to move the optometry unit 2 larger (or faster) than the fine movement in order to roughly adjust the relative position between the subject eye E and the optometry unit 2. In the present embodiment, the control unit 50 controls the optometry unit 2 to move larger than the fine movement, by allowing the optometry unit 2 to move within a movable range (within a movable range corresponding to a tiltable range of the operation stick 61) which is larger than a movable range for the fine movement. That is, the control unit 50 of the present embodiment enables the optometry unit 2 to move larger than the fine movement, by continuing the movement of the optometry unit 2 while an examiner inputs a direction for the rough movement, regardless of the movable range for the fine movement.

### (Operation Unit of Second Embodiment)

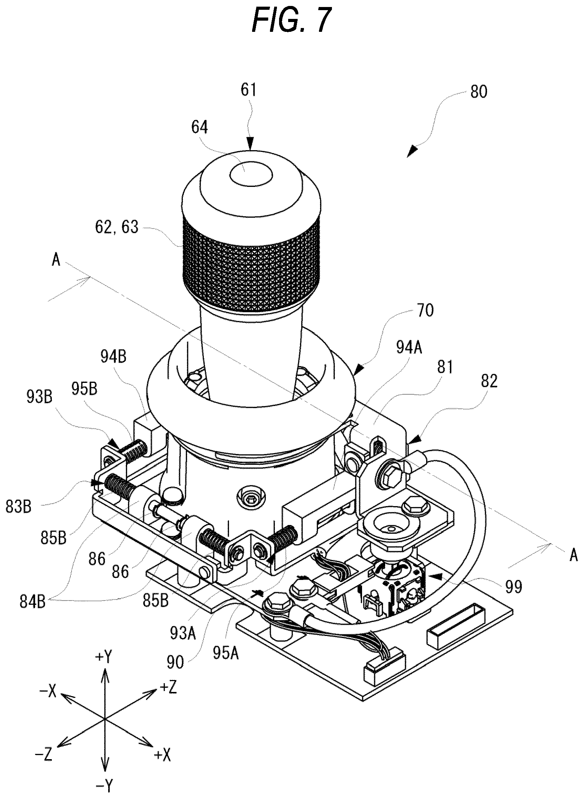

The operation unit 80 of the second embodiment will be described with reference to FIGS. 7 to 10. The same configuration as the configuration of the operation unit 60 of the first embodiment described above can be adopted for at least a part of the configuration of the operation unit 80 of the second embodiment. Therefore, in the following description, portions of the operation unit 80 of the second embodiment that can adopt the same configuration as the configuration of the operation unit 60 of the first embodiment are given the same numbers as in the first embodiment, and the description thereof will be omitted or simplified.

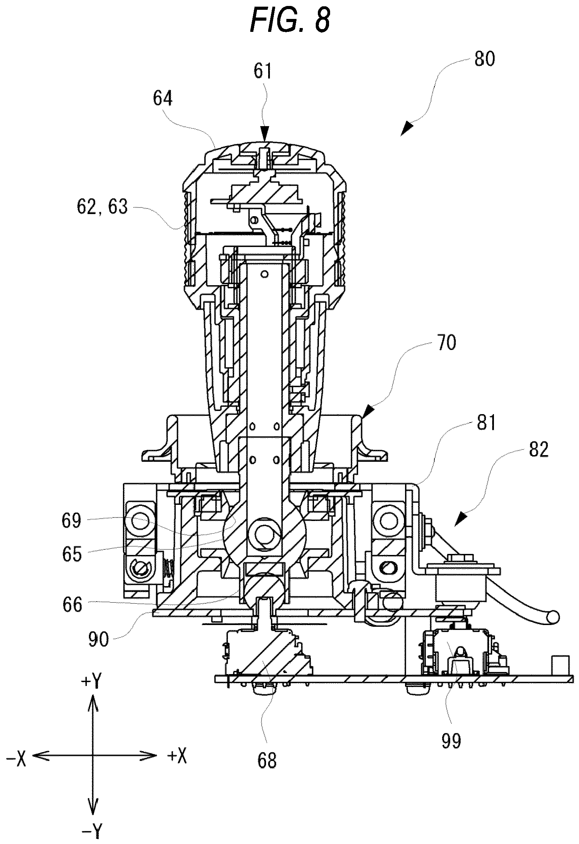

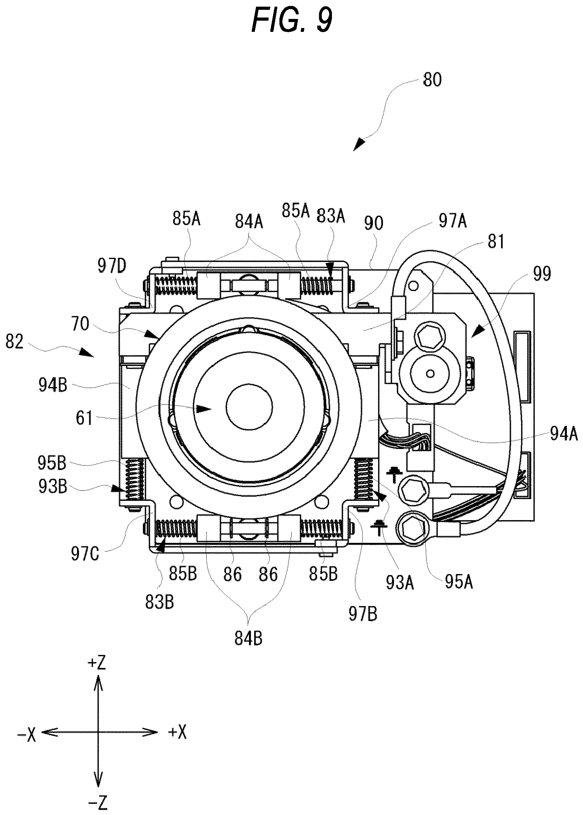

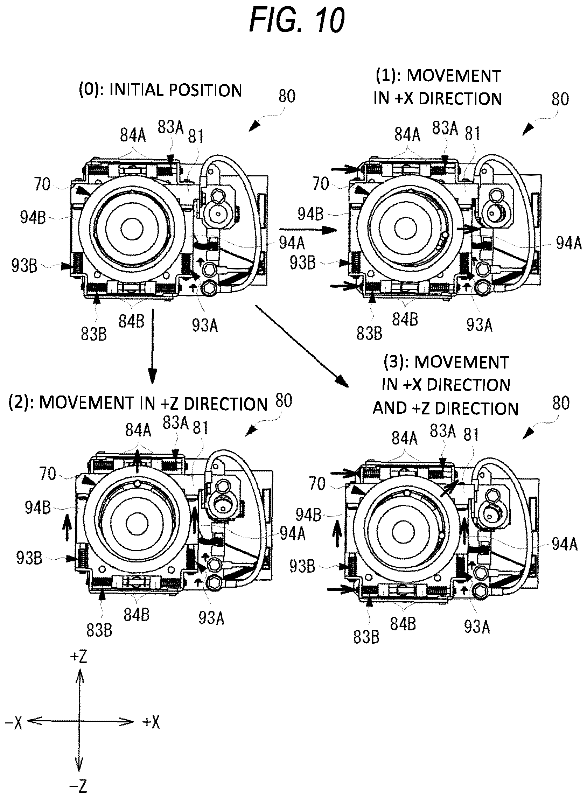

FIG. 7 is a perspective view of the operation unit 80 according to the second embodiment as viewed diagonally from the front right. FIG. 8 is a cross-sectional view taken along the line A-A in FIG. 7. FIG. 9 is a plan view of the operation unit 80 according to the second embodiment. FIG. 10 is a view comparing plan views of the operation unit 80 according to the second embodiment in cases where (0) the rough movement operation unit 70 is disposed at an initial position, (1) the rough movement operation unit 70 is moved in +X direction, (2) the rough movement operation unit 70 is moved in +Z direction, and (3) the rough movement operation unit 70 is moved in +X direction and +Z direction. In FIG. 7, the right front side of the paper is defined as +X direction, the left rear side of the paper is defined as -X direction, the upper side of the paper is defined as +Y direction, the lower side of the paper is defined as -Y direction, the right rear side of the paper is defined as +Z direction, and the left front side of the paper is defined as -Z direction. The X direction is one direction on a two-dimensional plane (slide plane) on which the rough movement operation unit 70 slides. The Z direction is a direction that intersects (perpendicularly intersects in the present embodiment) the X direction on the slide plane of the rough movement operation unit 70. The Y direction is a direction that intersects (perpendicularly intersects in the present embodiment) the slide plane of the rough movement operation unit 70. However, it goes without saying that each of the directions of XYZ can be appropriately changed.

The operation unit 80 of the second embodiment is also operated by the examiner to input an instruction to move the optometry unit 2 of the ophthalmic apparatus 1 to the ophthalmic apparatus 1, similar to the operation unit 60 of the first embodiment. When the operation unit 80 is operated, the control unit 50 outputs a signal corresponding to the content of the operation to the drive unit 4 to change the relative position of the optometry unit 2 with respect to the subject eye E. The operation unit 80 of the second embodiment is provided in the housing 3 (refer to FIG. 1) of the ophthalmic apparatus 1. However, the operation unit 80 may be provided independently of the housing 3 of the ophthalmic apparatus 1.

As illustrated in FIGS. 7 to 9, the operation unit 80 is provided with an operation stick (sometimes referred to as a joystick) 61 and the rough movement operation unit 70. In addition, the operation unit 80 is provided with an operation detection unit that detects operations of the operation stick 61 and the rough movement operation unit 70. The operation detection unit of the second embodiment includes a detector having the tilt detection unit 68 (refer to FIG. 8) that detects a tilt of the operation stick 61, and a rough movement operation detection unit 99 (refer to FIGS. 7 to 9) that detects operation of the rough movement operation unit 70. Since the operation stick 61, the grip unit 62, the rotary dial 63, the measurement button 64, the ball unit 65 (refer to FIG. 8), the bearing unit 69 (refer to FIG. 8), the action unit 66 (refer to FIG. 8), the tilt detection unit 68, and the like of the second embodiment can adopt the same configuration as that of the operation stick 61 of the first embodiment, detailed description thereof will be omitted.

In the second embodiment, similarly to the first embodiment, when a tilt operation of tilting the operation stick 61 within a predetermined range is detected, the position of the optometry unit 2 is finely changed in response to the detected tilt operation. When a tilt operation of tilting the operation stick 61 over a predetermined range is detected, the position of the optometry unit 2 is roughly changed in response to the detected tilt operation. In addition, when the operation of the rough movement operation unit 70 is detected, the position of the optometry unit 2 is roughly changed in response to the detected operation (according to the operation direction of the rough movement operation unit 70). That is, in the ophthalmic apparatus 1 of the second embodiment, the examiner can input a rough movement instruction for the optometry unit 2 even when either a tilt operation of tilting the operation stick 61 over a predetermined range or an operation of the rough movement operation unit 70 is performed.

As illustrated in FIGS. 7 to 10, the rough movement operation unit 70 of the second embodiment is an annular (substantially toric in the present embodiment) member disposed to surround the outer periphery of the operation stick 61 formed substantially in a rod shape. The rough movement operation unit 70 is supported so as to be slidable in two-dimensional directions on a slide plane which is the XZ plane. Therefore, the examiner can input the rough movement instruction for the optometry unit 2 in an appropriate direction by sliding the rough movement operation unit 70 around the operation stick 61 in the same direction as the tilt direction of the operation stick 61 in a case where the optometry unit 2 is moved, without performing the tilt operation of tilting the operation stick 61 over the predetermined range.

A rough movement operation interlocking unit 81 is connected with the rough movement operation unit 70. The rough movement operation interlocking unit 81 moves in conjunction with the operation of the rough movement operation unit 70 (sliding operation on the slide plane which is the XZ plane). As an example, the rough movement operation interlocking unit 81 of the second embodiment is configured by bending and connecting a substantially plate-like member. When the rough movement operation unit 70 slides on the XZ plane, the rough movement operation interlocking unit 81 also slides on the XZ plane. As illustrated in FIGS. 7 to 9, the rough movement operation detection unit 99 is provided in the rough movement operation interlocking unit 81. The rough movement operation detection unit 99 detects the operation direction of the rough movement operation unit 70 connected with the rough movement operation interlocking unit 81 by detecting the moving direction of the rough movement operation interlocking unit 81 on the XZ plane. In the second embodiment, the term "detection of operation of the rough movement operation unit 70" includes not only the detection of a direct operation of the rough movement operation unit 70 by the examiner, but also the detection of the tilt operation of tilting the operation stick 61 over a predetermined range. That is, even in a case where the tilt angle of the operation stick 61 exceeds the predetermined range and comes into contact with the annular rough movement operation unit 70 and the rough movement operation unit 70 slides in the XZ directions, the operation direction of the rough movement operation unit 70 is appropriately detected by the rough movement operation detection unit 99.

As illustrated in FIGS. 7 to 9, the operation unit 80 of the second embodiment is provided with a guide unit 82 that guides the movement of the rough movement operation unit 70 on the slide plane. Hereinafter, the configuration of the guide unit 82 of the second embodiment will be described in detail.

As illustrated in FIG. 9, the guide unit 82 is provided with a pair (two) of X-direction linear guide units 83A and 83B. The pair of X-direction linear guide units 83A and 83B linearly extend in the X direction while being parallel to each other. The X-direction linear guide unit 83A is disposed at the +Z direction side than the central portion of the rough movement operation unit 70 in the XZ direction. In addition, the X-direction linear guide unit 83B is disposed at the -Z direction side than the central portion of the rough movement operation unit 70 in the XZ direction. That is, the pair of X-direction linear guide units 83A and 83B are disposed so as to interpose the central portion of the rough movement operation unit 70 in the XZ direction.

In addition, the guide unit 82 is provided with a pair (two) of Z-direction linear guide units 93A and 93B. The pair of Z-direction linear guide units 93A and 93B linearly extend in the Z direction while being parallel to each other. The Z-direction linear guide unit 93A is disposed at the +X direction side than the central portion of the rough movement operation unit 70 in the XZ direction. In addition, the Z-direction linear guide unit 93B is disposed at the -X direction side than the central portion of the rough movement operation unit 70 in the XZ direction. That is, the pair of Z-direction linear guide units 93A and 93B are disposed so as to interpose the central portion of the rough movement operation unit 70 in the XZ direction.

In the second embodiment, a guide shaft is used for each of the four linear guide units 83 and 93 (that is, the pair of X-direction linear guide units 83A and 83B and the pair of Z-direction linear guide units 93A and 93B). However, at least one of the four linear guide units 83 and 93 can adopt a configuration different from that of the guide shaft. For example, a guide rail or the like may be adopted instead of the guide shaft.

As illustrated in FIG. 9, the guide unit 82 is provided with a pair (two) of X-direction slide units 84A and 84B. The X-direction slide unit 84A is attached to the X-direction linear guide unit 83A and has a slider which relatively slides in the X direction with respect to the X-direction linear guide unit 83A. The X-direction slide unit 84B is attached to the X-direction linear guide unit 83B and has a slider which relatively slides in the X direction with respect to the X-direction linear guide unit 83B.

In addition, the guide unit 82 is provided with a pair (two) of Z-direction slide units 94A and 94B. The Z-direction slide unit 94A is attached to the Z-direction linear guide unit 93A and has a slider which relatively slides in the Z direction with respect to the Z-direction linear guide unit 93A. The Z-direction slide unit 94B is attached to the Z-direction linear guide unit 93B and has a slider which relatively slides in the Z direction with respect to the Z-direction linear guide unit 93B.

In the second embodiment, bearings through which guide shafts are inserted are used for the four slide units 84 and 94 (that is, the pair of X-direction slide units 84A and 84B and the pair of Z-direction slide units 94A and 94B). However, at least one of the four slide units 84 and 94 can adopt a configuration different from that of the bearing. For example, instead of bearings, a carriage or the like that relatively moves along guide rails may be adopted.

As illustrated in FIG. 10, according to the guide unit 82 of the second embodiment, the pair of X-direction linear guide units 83A and 83B slide in the X direction with respect to the pair of X-direction slide units 84A and 84B to guide the movement of the rough movement operation unit 70 in the X direction (refer to case (0) and case (1) in FIG. 10). In addition, the pair of Z-direction slide units 94A and 94B slide in the Z direction with respect to the pair of Z-direction linear guide units 93A and 93B to guide the movement of the rough movement operation unit 70 in the Z direction (refer to case (0) and case (2) in FIG. 10). That is, the X-direction linear guide units 83A and 83B slide with respect to the X-direction slide units 84A and 84B, while the Z-direction slide units 94A and 94B slide with respect to the Z-direction linear guide units 93A and 93B. As a result, while adopting a similar configuration (configuration using a linear guide unit and a slide unit) in each of the +X direction, -X direction, +Z direction, and -Z direction, in addition to the movement of the rough movement operation unit 70 in the X direction and the Z direction, a movement in an oblique direction inclined to both the X and Z directions is also appropriately guided (refer to case (0) and case (3) in FIG. 10).

In addition, in the second embodiment, it is easy to reduce the size of the guide unit 82 in the Y direction by adopting a configuration in which the slide units 84 and 94 are attached respectively to the four linear guide units 83 and 93 to the guide unit 82. In particular, in the guide unit 82 of the second embodiment, the pair of X-direction linear guide units 83A and 83B and the pair of Z-direction linear guide units 93A and 93B (that is, the four linear guide units 83 and 93) are disposed on the same XZ plane. Therefore, it is easier to reduce the size of the guide unit 82 in the Y direction.

The four linear guide units 83 and 93 and the four slide units 84 and 94 in the second embodiment are disposed at each side of the +X direction, -X direction, +Z direction, and -Z direction than the central portion so as to surround the central portion of the rough movement operation unit 70 in the XZ direction. Therefore, the load applied from the rough movement operation unit 70 to the guide unit 82 can be easily distributed, as compared to the case where the guide unit 82 is disposed biasedly in either direction with respect to the central portion of the rough movement operation unit 70. Therefore, the movement of the rough movement operation unit 70 on the XZ plane is easily guided more smoothly. In particular, in the guide unit 82 of the second embodiment, the four linear guide units 83 and 93 are disposed at positions that are four-fold rotationally symmetrical about the center of the rough movement operation unit 70 in the XZ direction. As a result, the load applied from the rough movement operation unit 70 to the guide unit 82 is easily distributed more evenly. Therefore, the movement of the rough movement operation unit 70 on the XZ plane is easily guided more smoothly.

As illustrated in FIG. 9, the pair of X-direction linear guide units 83A and 83B is provided respectively with X-direction biasing units 85A and 85B. The X-direction biasing units 85A and 85B biases the positions of the X-direction linear guide units 83A and 83B with respect to the X-direction slide units 84A and 84B toward the initial X position (the position of the rough movement operation unit 70 illustrated in FIGS. 9 and 10) in a state where the rough movement operation unit 70 is not operated in the X direction. Therefore, the position of the rough movement operation unit 70 after the rough movement operation in the X direction is completed is likely to be automatically returned to the initial X position by the X-direction biasing units 85A and 85B.

In the present embodiment, when the position of the rough movement operation unit 70 is the initial X position, distances between the left end of a connecting unit 97A (details will be described later) in FIG. 9 and the right end of the X-direction slide unit 84A, and between the right end of a connecting unit 97D (details will be described later) and the left end of the X-direction slide unit 84A are equal to each other. The X-direction biasing unit 85A (in the present embodiment, a spring inserted through the X-direction linear guide unit 83A) is provided at each of the spaces between the left end of the connecting unit 97A and the right end of the X-direction slide unit 84A, and between the right end of the connecting unit 97D and the left end of the X-direction slide unit 84A. The biasing forces of the two X-direction biasing units 85A are substantially the same. Similarly, in a case where the position of the rough movement operation unit 70 is the initial X position, distances between the left end of a connecting unit 97B (details will be described later) and the right end of the X-direction slide unit 84B, and between the right end of a connecting unit 97C and the left end of the X-direction slide unit 84B are equal to each other. The X-direction biasing unit 85B (in the present embodiment, a spring inserted through the X-direction linear guide unit 83B) is provided at each of the spaces between the left end of the connecting unit 97B and the right end of the X-direction slide unit 84B, and between the right end of the connecting unit 97C and the right end of the X-direction slide unit 84B. The biasing forces of the two X-direction biasing units 85B are substantially the same. With the above configuration, the position of the rough movement operation unit 70 is likely to be more appropriately returned to the initial X position.

In addition, Z-direction biasing units 95A and 95B are provided respectively on the pair of Z-direction linear guide units 93A and 93B. The Z-direction biasing units 95A and 95B biases the positions of the Z-direction slide units 94A and 94B with respect to the Z-direction linear guide units 93A and 93B toward the initial Z position in a state where the rough movement operation unit 70 is not operated in the Z direction. Therefore, the position of the rough movement operation unit 70 after the rough movement operation in the Z direction is completed is likely to be automatically returned to the initial Z position by the Z-direction biasing units 95A and 95B.

In the present embodiment, in a case where the position of the rough movement operation unit 70 is the initial Z position, distances between the lower end of the connecting unit 97A (details will be described later) in FIG. 9 and the upper end of the Z-direction slide unit 94A, and between the upper end of the connecting unit 97B (details will be described later) and the lower end of the Z-direction slide unit 94A are equal to each other. The Z-direction biasing unit 95A (in the present embodiment, a spring inserted through the Z-direction linear guide unit 93A) is provided at each of the spaces between the lower end of the connecting unit 97A and the upper end of the Z-direction slide unit 94A, and between the upper end of the connecting unit 97B and the lower end of the Z-direction slide unit 94A. In FIG. 9, the Z-direction biasing unit 95A in the +Z direction of the two Z-direction biasing units 95A is hidden by the rough movement operation interlocking unit 81. The biasing forces of the two Z-direction biasing units 95A are substantially the same. Similarly, in a case where the position of the rough movement operation unit 70 is the initial Z position, distances between the lower end of the connecting unit 97D (details will be described later) in FIG. 9 and the upper end of the Z-direction slide unit 94B, and between the upper end of the connecting unit 97C (details will be described later) and the lower end of the Z-direction slide unit 94B are equal to each other. The Z-direction biasing unit 95B (in the present embodiment, a spring inserted through the Z-direction linear guide unit 93B) is provided at each of the spaces between the lower end of the connecting unit 97D and the upper end of the Z-direction slide unit 94B, and between the upper end of the connecting unit 97C and the lower end of the Z-direction slide unit 94B. In FIG. 9, the Z-direction biasing unit 95B in the +Z direction of the two Z-direction biasing units 95B is hidden by the rough movement operation interlocking unit 81. The biasing forces of the two Z-direction biasing units 95B are substantially the same. With the above configuration, the position of the rough movement operation unit 70 is likely to be more appropriately returned to the initial Z position.

As illustrated in FIG. 9, the X-direction linear guide unit 83B is provided with an X-direction movement amount regulating unit 86. When the X-direction linear guide unit 83B reaches the limits of movable ranges in each of the +X direction and the -X direction, the X-direction movement amount regulating unit 86 contacts the X-direction slide unit 84B. As a result, the amount of movement of the rough movement operation unit 70 in the X direction is appropriately regulated by the X-direction movement amount regulating unit 86. It goes without saying that the X-direction linear guide unit 83A may also be provided with a similar X-direction movement amount regulating unit.

In addition, when the Z-direction slide units 94A and 94B reach the limits of their movable ranges in each of the +Z direction and the -Z direction, the Z-direction slide units 94A and 94B come into contact with the connecting units 97A, 97B, 97C, and 97D (details will be described later). As a result, the amount of movement of the rough movement operation unit 70 in the Z direction is appropriately regulated.

As illustrated in FIG. 7, in the present embodiment, since the rough movement operation unit 70 is connected with each of the pair of Z-direction slide units 94A and 94B, the pair of Z-direction slide units 94A and 94B also move on the XZ plane in synchronization with the movement of the rough movement operation unit 70 on the XZ plane. As an example, the rough movement operation unit 70 of the present embodiment is connected with each of the pair of Z-direction slide units 94A and 94B via the rough movement operation interlocking unit 81. In addition, in the present embodiment, the four linear guide units 83 and 93 (that is, a pair of X-direction linear guide units 83A and 83B and a pair of Z-direction linear guide units 93A and 93B) are connected with each other to integrally move in the X direction. As illustrated in FIG. 9, in the present embodiment, the +X direction side of the X-direction linear guide unit 83A and the +Z direction side of the Z-direction linear guide unit 93A are connected by the connecting unit 97A. The -Z direction side of the Z-direction linear guide unit 93A and the +X direction side of the X-direction linear guide unit 83B are connected by the connecting unit 97B. The -X direction side of the X-direction linear guide unit 83B and the -Z direction side of the Z-direction linear guide unit 93B are connected by the connecting unit 97C. The +Z direction side of the Z-direction linear guide unit 93B and the -X direction side of the X-direction linear guide unit 83A are connected by the connecting unit 97D. In addition, the pair of X-direction slide units 84A and 84B is fixed to a base unit 90 (refer to FIGS. 7 to 9) which is a base.

The operation of the guide unit 82 having the above configuration will be described. As illustrated in case (0) and case (2) in FIG. 10, when the rough movement operation unit 70 is moved in the Z direction, the pair of Z-direction slide units 94A and 94B connected with the rough movement operation unit 70 also moves in the Z direction. As a result, since the pair of Z-direction slide units 94A and 94B slide in the Z direction with respect to the pair of Z-direction linear guide units 93A and 93B, the movement of the rough movement operation unit 70 in the Z direction is appropriately guided. In addition, as illustrated in case (0) and case (1) in FIG. 10, when the rough movement operation unit 70 is moved in the X direction, since the pair of Z-direction slide units 94A and 94B also move in the X direction, the four linear guide units 83 and 93 connected with each other also move in the X direction together with the Z-direction slide units 94A and 94B. When the four linear guide units 83 and 93 integrally move in the X direction, since the pair of X-direction linear guide units 83A and 83B move in the X direction in synchronization with the pair of X-direction slide units 84A and 84B, the movement of the rough movement operation unit 70 in the X direction is also appropriately guided. Therefore, the movement of the rough movement operation unit 70 on the XZ plane is appropriately guided (refer to case (3) in FIG. 10).

The techniques disclosed in the above embodiments are merely examples. Therefore, it is possible to modify the techniques exemplified in the above embodiments. For example, when the tilt angle exceeds a predetermined range, the operation stick 61 of the above embodiment is tilted while sliding the rough movement operation unit 70 by contacting the annular rough movement operation unit 70. However, even when the configuration in which the operation stick 61 and the rough movement operation unit 70 are brought into contact with each other is not adopted, it is also possible to accept an input of the rough movement instruction by both the tilt operation of tilting the operation stick 61 over a predetermined range and the operation of the rough movement operation unit 70. For example, the tilt detection unit 68 may detect that the tilt angle of the operation stick 61 exceeds a predetermined range. In addition, in a case where the tilt angle of the operation stick 61 exceeds a predetermined range, a member that moves in conjunction with the tilt operation of tilting the operation stick 61 may be separately detected by a detection unit (sensor or the like).

In addition, the operation unit 80 exemplified in the second embodiment is provided with the pair (two) of X-direction linear guide units 83A and 83B and the pair of X-direction slide units 84A and 84B. However, it is also possible to set the number of each of the X-direction linear guide unit and the X-direction slide unit to one. In addition, the operation unit 80 exemplified in the second embodiment is provided with the pair (two) of Z-direction linear guide units 93A and 93B and the pair of Z-direction slide units 94A and 94B. However, it is also possible to set the number of each of the Z-direction linear guide unit and the Z-direction slide unit to one. Even in these cases, the guide unit of the present disclosure can be easily installed in a narrow space and can smoothly guide the movement of the rough movement operation unit.

The processing of finely moving the position of the optometry unit 2 in S4 and S5 in FIG. 6 is an example of fine driving of the drive unit 4 under the control of the control unit 50. The processing of roughly moving the position of the optometry unit 2 in S7 and S8 of FIG. 6 is an example of rough driving of the drive unit 4 under the control of the control unit 50.

## Claims

1. An ophthalmic apparatus comprising:
an optometry unit configured to examine a subject eye;
a drive unit configured to change a relative position of the optometry unit with respect to the subject eye;
an operation stick supported to be tiltable in any direction;
a rough movement operation unit operated by an examiner to roughly move the optometry unit;
an operation detection unit configured to detect an operation of the operation stick and the rough movement operation unit; and
a control unit configured to:
control the drive unit, in a case where the operation detection unit detects a tilt operation of tilting the operation stick within a predetermined range, in response to the detected tilt operation, to finely drive the drive unit and finely change a position of the optometry unit; and
control the drive unit, in a case where the operation detection unit detects at least one of a tilt operation of tilting the operation stick over the predetermined range and an operation of the rough movement operation unit, in response to the detected operation, to roughly drive the drive unit and roughly change the position of the optometry unit.

2. The ophthalmic apparatus according to claim 1,
wherein the rough movement operation unit is an annular member disposed on an outer periphery of the operation stick formed in a rod shape to surround the operation stick, and
wherein the rough movement operation unit is supported to be slidable in two-dimensional directions.

3. The ophthalmic apparatus according to claim 1 or 2,
wherein in a case where a tilt angle of the operation stick is within the predetermined range, the operation stick is tilted to be independent of the rough movement operation unit, and
wherein in a case where the tilt angle of the operation stick exceeds the predetermined range, the operation stick is tilted while contacting the rough movement operation unit to operate the rough movement operation unit.

4. The ophthalmic apparatus according to claim 3,
wherein the operation detection unit includes a rough movement operation detection unit that detects that the rough movement operation unit is operated, and
wherein both the tilt operation of tilting the operation stick over the predetermined range and the operation of the rough movement operation unit are detected by the rough movement operation detection unit.

5. The ophthalmic apparatus according to claim 3 or 4, further comprising:
a biasing unit configured to bias the rough movement operation unit toward an initial position where the rough movement operation unit is in contact with the operation stick in a state where the tilt angle reaches a boundary of the predetermined range,
wherein when the tilt operation of tilting the operation stick over the predetermined range is completed, the biasing unit moves the rough movement operation unit toward the initial position to return the tilt angle of the operation stick being in contact with the rough movement operation unit within the predetermined range.

6. The ophthalmic apparatus according to any one of claims 1 to 5,
wherein in a case where an operation amount of the rough movement operation unit exceeds a specified amount, the operation detection unit detects that the rough movement operation unit is operated.

7. The ophthalmic apparatus according to any one of claims 1 to 6, further comprising:
a guide unit configured to guide a movement of the rough movement operation unit on a slide plane which is a two-dimensional plane on which the rough movement operation unit is slid, wherein one direction on the slide plane is defined as an X direction, a direction intersecting the X direction on the slide plane is defined as a Z direction, and a direction intersecting the slide plane is defined as a Y direction,
wherein the guide unit includes:
an X-direction linear guide unit linearly extending in the X direction;
a Z-direction linear guide unit linearly extending in the Z direction;
an X-direction slide unit attached to the X-direction linear guide unit and relatively sliding in the X direction with respect to the X-direction linear guide unit; and
a Z-direction slide unit attached to the Z-direction linear guide unit and relatively sliding in the Z direction with respect to the Z-direction linear guide unit,
wherein the X-direction linear guide unit slides in the X direction with respect to the X-direction slide unit to guide a movement of the rough movement operation unit in the X direction, and
wherein the Z-direction slide unit slides in the Z direction with respect to the Z-direction linear guide unit to guide a movement of the rough movement operation unit in the Z direction.

8. The ophthalmic apparatus according to claim 7,
wherein a pair of the X-direction linear guide units are disposed at each side of a +Z direction and a -Z direction than a central portion in an XZ direction of the rough movement operation unit, and extend linearly in the X direction in parallel to each other,
wherein a pair of the Z-direction linear guide units are disposed at each side of a +X direction and a -X direction than the central portion in the XZ direction of the rough movement operation unit, and extend linearly in the Z direction in parallel to each other,
wherein a pair of the X-direction slide units are attached to each of the pair of X-direction linear guide units, and
wherein a pair of the Z-direction slide units are attached to each of the pair of Z-direction linear guide units.

9. The ophthalmic apparatus according to claim 7 or 8,
wherein the X-direction linear guide unit is provided with an X-direction biasing unit configured to bias a position of the X-direction linear guide unit with respect to the X-direction slide unit toward an initial X position where the rough movement operation unit is not operated in the X direction, and
wherein the Z-direction slide unit is provided with a Z-direction biasing unit configured to bias a position of the Z-direction slide unit with respect to the Z-direction linear guide unit toward an initial Z position where the rough movement operation unit is not operated in the Z direction.

10. The ophthalmic apparatus according to any one of claims 7 to 9,
wherein the X-direction linear guide unit and the Z-direction linear guide unit are disposed on the same XZ plane.

11. The ophthalmic apparatus according to any one of claims 7 to 10,
wherein the rough movement operation unit is connected with the Z-direction slide unit, in which the Z-direction slide unit moves on an XZ plane in synchronization with a movement of the rough movement operation unit on the XZ plane, and
wherein the X-direction linear guide unit and the Z-direction linear guide unit are connected with each other to integrally move in the X direction.

12. An operation unit operated by an examiner to change a relative position of an optometry unit of an ophthalmic apparatus with respect to a subject eye, the operation unit comprising:
an operation stick supported to be tiltable in any direction;
a rough movement operation unit operated by the examiner to roughly move the optometry unit; and
an operation detection unit configured to detect an operation of the operation stick and the rough movement operation unit,
wherein in a case where the operation detection unit detects a tilt operation of tiling the operation stick within a predetermined range, a position of the optometry unit is finely changed in response to the detected tilt operation, and
wherein in a case where the operation detection unit detects at least one of a tilt operation of tiling the operation stick over the predetermined range and an operation of the rough movement operation unit, the position of the optometry unit is roughly changed in response to the detected operation.
